(19) Europäisches Patentamt
European Patent Office
Office européen des brevets

(11) Veröffentlichungsnummer: **0 150 320 B1**

(12) **EUROPÄISCHE PATENTSCHRIFT**

(45) Veröffentlichungstag der Patentschrift: **23.05.90**

(51) Int. Cl.⁵: **F 16 C 39/06, A 61 M 1/10**

(21) Anmeldenummer: **84114324.1**

(22) Anmeldetag: **27.11.84**

(54) **Magnetische Rotorlagerung.**

(30) Priorität: **29.11.83 DE 3343186**

(43) Veröffentlichungstag der Anmeldung:
**07.08.85 Patentblatt 85/32**

(45) Bekanntmachung des Hinweises auf die
Patenterteilung:
**23.05.90 Patentblatt 90/21**

(84) Benannte Vertragsstaaten:
**AT CH DE FR GB IT LI NL SE**

(56) Entgegenhaltungen:
**EP-A-0 060 569**
**EP-A-00 019 313**
**DE-A-2 336 488**
**DE-A-3 130 974**
**FR-A-2 165 633**

(73) Patentinhaber: **FRAUNHOFER-GESELLSCHAFT ZUR FÖRDERUNG DER ANGEWANDTEN FORSCHUNG E.V.**
**Leonrodstrasse 54**
**D-8000 München 19 (DE)**

(72) Erfinder: **Bramm, Gunter**
**Luisenstrasse 49**
**D-8000 München 40 (DE)**
Erfinder: **Novak, Pavel, Dr.**
**Schleissheimer Strase 44**
**D-8000 München 2 (DE)**

(74) Vertreter: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

EP 0 150 320 B1

**Beschreibung**

Die Erfindung betrifft eine magnetische Rotorlagerung zum berührungslosen Aufhängen eines Rotors, insbesondere des Rotors einer Axial- oder Radial-Zentrifugalblutpumpe, mit einer den Rotor in einem Stator, insbesondere einem Gehäuse, aufhängenden und lagestabilisierenden Permanent- und Elektromagnetanordnung, sowie mit einer an wenigstens einen Lagesensor bzw. dessen Lagesensorbetriebsschaltung für den Rotor angeschlossenen Regelschaltung, wobei die Permanent- und Elektromagnetanordnung so ausgebildet ist, daß

(a) der Rotor mittels einer Permanentmagnetanordnung, welche wenigstens einen im Rotor und wenigstens einen im Stator angebrachten axial magnetisierten Permanentmagneten umfaßt, bis auf einen einzigen geometrischen Verstellfreiheitsgrad stabil berührungslos im Stator aufgehängt ist;

(b) die Rotorposition nur innerhalb des mittels der Permanentmagnetanordnung nicht stabilisierten geometrischen Verstellfreiheitsgrads mittels einer im Stator vorgesehenen, wenigstens einen axialen Elektromagneten umfassenden Elektromagnetanordnung und einer damit zusammenwirkenden, im Rotor befindlichen und wenigstens einen axial magnetisierten Permanentmagneten umfassenden Permanentmagnetanordnung stabilisiert ist; und

(c) die mit der Elektromagnetanordnung zusammenwirkende Permanentmagnetanordnung wenigstens einen Permanentmagneten der zum Aufhängen des Rotors vorgesehenen Permanentmagnetanordnung umfaßt, insbesondere aus den im Rotor angebrachten Permanentmagneten dieser permanentmagnetanordnung besteht.

Obwohl die magnetische Rotorlagerung nach der Erfindung für den Rotor einer Axial- oder Radial-Zentrifugalblutpumpe eines künstlichen Herzens entwickelt worden ist, ist ihre Anwendung keineswegs auf derartige Zentrifugalblutpumpen beschränkt, sondern sie ist darüber hinaus für die verschiedensten Rotoren geeignet, die mittels einer berührungslosen magnetischen Aufhängung gelagert werden können. So ist die magnetische Rotorlagerung einschließlich der zugehörigen, an einen oder mehrere Lagesensoren angeschlossenen Schaltungsanordnung zum Einstellen der Magnetkraft der darin vorgesehenen Elektromagnetanordnung auch für die Kreiselkompaßlagerung und den Kreiselkompaßantrieb, beispielsweise für deren Verwendung in der Raumfahrttechnik, beim Flugbetrieb und in U-Booten o.dgl., sowie für Kreiselrichtungsstabilisatoren, die zum Beispiel in raketengetriebenen Flugkörpern vorgesehen werden und auch für Zeigerinstrumente o.dgl. geeignet.

Auch hinsichtlich ihrer Anwendung in Pumpen ist die mit der Erfindung zur Verfügung gestellte magnetische Rotorlagerung nicht auf Axial- oder Radial-Zentrifugalblutpumpen beschränkt, sondern sie läßt sich auch bei anderen Blutpumpen anwenden, die einen Rotor besitzen, der magnetisch aufgehängt werden kann. Weiterhin kann die magnetische Rotorlagerung auch bei anderen Rotorpumpen angewandt werden; zum Beispiel kann sie mit besonderem Vorteil in Rotorpumpen für radioaktive Flüssigkeiten verwendet werden, weil solche Rotorpumpen, wenn sie mit einer magnetischen Rotorlagerung nach der Erfindung versehen sind, reibungs- und damit wartungsfrei sind, da sie keine mechanischen Lager, keine Ventile, keine Dichtungen etc. benötigen, die einem Verschleiß unterliegen.

Magnetische Rotorlagerungen zum berührungslosen Aufhängen eines Rotors sind an sich in verschiedenen Ausführungsformen bekannt. So ist aus der US-Patentschrift 3 938 913 eine Strömungseinrichtung zum Pumpen und/oder Messen der Strömung von aggressiven, radioaktiven oder besonders reinen Strömungsmitteln bekannt, in der ein Rotor berührungslos magnetisch in einem Gehäuse aufgehängt ist, wobei zur Aufhängung des Rotors Elektromagnete in dem Gehäuse vorgesehen sind, die mit magnetischem Material, das gegenüber den Elektromagneten im Rotor angebracht ist, jeweils einen magnetischen Kreis bilden. In diesen magnetischen Kreisen werden nur anziehende Kräfte angewandt, durch welche der Rotor innerhalb des Gehäuses in der Schwebe gehalten wird.

Die Anwendung von ausschließlich anziehenden Kräften, die mit Elektromagneten erzeugt werden, hat jedoch erhebliche Nachteile:

(a) Die Stabilität der magnetischen Lagerung, d.h. die Stabilität der Aufrechterhaltung der richtigen Schwebelage des Rotors, bei welcher der Rotor in allen Radialrichtungen in einem etwa gleich großen Abstand von der Gehäuseinnenwand angeordnet ist, ist gegenüber eigenerregten und fremderregten Schwingungen sehr unbefriedigend. Eine derartige magnetische Rotorlagerung neigt wegen des verzögerten Magnetfeldaufbaus verhältnismäßig leicht zu Schwingungen. Dieser verzögerte Magnetfeldaufbau resultiert aus den relativ großen Induktivitäten, die erforderlich sind, um das gesamte Magnetfeld jeweils durch elektrische Ströme aufzubauen, und wegen der hohen Permeabilität von Eisen ($\mu$ relativ bis zu 10,000) ergeben sich hohe absolute Induktivitäten, die einen verzögerten Stromanstieg zur Folge haben.

(b) Der Energieverbrauch einer solchen Elektromagnete aufweisenden magnetischen Rotorlagerung ist sehr hoch, und gleichzeitig ist der Wirkungsgrad verhältnismäßig schlecht, so daß in hohem Maße unerwünschte Wärmeenergie entwickelt wird, die nicht nur nutzlos vergeudet wird und deren Abführung nicht nur schwierig ist, sondern die darüber hinaus bei Blutpumpen äußerst gefährlich ist, da das Bluteiweiß bei 42°C koaguliert.

(c) Die spezifischen Kräfte sind relativ gering, ebenso die relativen Spitzenkräfte, weil Eisen eine hohe Dichte hat und Felder wesentlich über 10 Kilogauß zu Sättigungserscheinungen führen, wobei $\mu$ relativ gegen 1 geht.

Weiterhin ist aus der europäischen Offenlegungsschrift 0 060 569 der europäischen Patentanmeldung 82 102 188.8 eine u.a. vom Erfinder der vorliegenden magnetischen Rotorlagerung früher entwickelte magnetische Rotorlagerung zum berührungslosen Aufhängen eines Rotors einer Zentrifugalblutpumpe bekannt, in welcher zur Aufhängung des Rotors eine kombinierte Elektromagnet- und Permanentmagnet-Anordnung vorgesehen ist, die aus Elektromagneten besteht, welche in dem das Gehäuse der Zentrifugalblutpumpe bildenden Stator vorgesehen sind, und aus Permanentmagneten, welche gegenüber den Elektromagneten im Rotor angebracht sind, so daß sie mit den Elektromagneten zusammenwirken.

Diese kombinierte Elektromagnet- und Permanentmagnet-Anordnung hat im Prinzip die gleichen Nachteile, wie sie vorstehend in Verbindung mit der magnetischen Rotorlagerung nach der US-Patentschrift 3 938 913 erwähnt sind. Denn die ausschließliche Anwendung von Elektromagneten im Stator führt zu einem verhältnismäßig hohen Energieverbrauch und damit zu starker Wärmeentwicklung, und außerdem ergibt sich eine relativ unbefriedigende Stabilität infolge des den Elektromagneten inhärenten verzögerten Feldaufbaus.

Weiterhin ist aus EP-A-0 019 313 eine magnetische Rotorlagerung der eingangs genannten Art bekannt, bei der sowohl im Stator als auch im Rotor Permanentmagnete angebracht sind, wobei mittig zwischen zwei axial im Abstand voneinander befindlichen Permanentmagneten im Stator und zwei in axialem Abstand voneinander angeordneten Permanentmagneten im Rotor ein Elektromagnet im Stator angebracht ist.

In dieser magnetischen Rotorlagerung sind die Permanentmagnete des Rotors im gleichen axialen Abstand voneinander angeordnet, wobei sich in axialer Richtung eine durch den Elektromagneten stabilisierte Gleichgewichtslage des Rotors ergibt, in welcher sich der jeweilige Permanentmagnet des Rotors axial mittig innerhalb des zugeordneten Permanentmagneten des Stators befindet. Aufgrund dieses Aufbaus ergibt sich eine maximal ungünstige Rotorzentrierungskraft in radialer Richtung, wobei sich im einzelnen folgende Nachteile ergeben:

(1) Für eine vorgegebene Permanentmagnetmaterialmenge wird nur die kleinstmögliche spezifische Kraftentwicklung für die Rückführung des Rotors in die radial mittige Lage erhalten, das heißt nur eine sehr geringe Lagersteifigkeit in Radialrichtung.

(2) Der Luftspalt zwischen dem jeweiligen Rotorpermanentmagnet und dem zugeordneten Statorpermanentmagnet muß verhältnismäßig groß sein und darf einen bestimmten Minimalwert nicht unterschreiten, da dann die Abstoßung zu Null wird oder sogar in eine Anziehung "umkippt". Dieser verhältnismäßig große erforderliche Luftspalt hat im einzelnen folgende Nachteile:

(a) Der Aufbau ist relativ raumaufwendig, was insbesondere bei Blutpumpen, die implantiert werden sollen, sehr nachteilig ist.

(b) Die obengenannte, zum Zentrieren des Rotors verfügbare Kraft läßt sich durch Verkleinerung des Luftspalts nicht erhöhen, sondern nimmt aufgrund der zentrierten axialen Lage der Permanentmagnete sogar ein relatives Minimum an.

(c) Es ergibt sich ein verhältnismäßig hohes Rotorgewicht aufgrund der für die radiale Magnetkraft erforderlichen, relativ hohen Permanentmagnetmaterialmenge, was wiederum folgende Nachteile hat:

($\propto$) Der Kraftanteil, der zum Tragen des im Rotor untergebrachten Magnetlagergewichts erforderlich ist, ist ziemlich hoch;

($\beta$) der Kraftanteil, der für das Tragen des übrigen Rotorgewichts, das heißt der Nutzlast, zur Verfügung steht, wird entsprechend klein; und

($\gamma$) der Kraftanteil, der zur laufende Rückführung des Rotors in die radial zentrale Lage erforlich ist, wird ebenfalls entsprechend klein (geringe Lagersteifigkeit).

Die obigen Nachteile des Magnetlagers nach EP-A-0 019 313 erbringen jedoch keineswegs Vorteile bezüglich der axialen Stabilität, sondern vielmehr ist das axiale Lagegleichgewicht des Rotors ohne elektromagnetische Stabilisierung auch labil wie bei der vorliegenden Erfindung und, darüber hinaus ist es bei dem Magnetlager nach EP-A-0 019 313 sogar so, daß die labilen spezifischen Kräfte, das heißt die den Rotor axial aus der Mitte drängenden Kräfte, größer als bei der erfindungsgemäßen Magnetlagerung sind, weil bei der Magnetlagerung nach EP-A-0 019 313 bereits beim Einbringen des Rotorpermanentmagneten in die mittige axiale Position des Statormagneten der größte Teil der vorhandenen Feldenergie durch Feldverdrängung gespeichert wurde, und dieser relativ große Teil wird beim Auslenken aus der axialen Mittenlage wieder frei.

Mit der Erfindung soll demgegenüber eine magnetische Rotorlagerung der eingangs genannten Art zur Verfügung gestellt werden, die insbesondere die nachfolgenden beiden Forderungen erfüllt:

(1) Die magnetische Rotorlagerung soll bei optimal radial zentrierender Kraftwirkung einen höchstmöglichen elektromechanischen Wirkungsgrad haben, damit der in Wärme umgewandelte Anteil der für die magnetische Aufhängung und Lagestabilisierung des Rotors aufgewandten Energie prozentual möglichst klein ist und damit die Betriebstemperatur auf möglichst niedrige Werte, insbesondere auf Werte beschränkt bleibt, die unterhalb der Temperatur liegen, bei welcher eine Koagulation des Bluteiweißes einsetzt.

(2) Der absolute Energiebedarf der magnetischen Rotorlagerung soll so klein wie möglich sein, damit der absolute Energiebetrag, der aufgrund der magnetischen Aufhängung und Stabilisierung des Rotors in Wärmeenergie umgewandelt wird, möglichst gering ist und damit außerdem jeder Baustein der gesamten magnetischen Rotorlagerung möglichst klein wird, was besonders für die Anwendung der magnetischen Rotorlagerung in einer Zentrifugalblutpumpe, welche implantiert werden soll, wichtig ist, insbesondere im Hinblick darauf, daß schon eine geringe Verminderung des Energiebedarfs der Zentrifugalblutpumpe selbst eine große Verminderung des Gewichts, des Volumens und des Leistungsbedarfs der gesamten zu implantierenden Zentrifugalblutpumpenanordnung zur Folge hat, welche außer der Zentrifugalblutpumpe eine Regelschaltung und beispielweise einen Energiewandler oder -speicher sowie eine induktive Energieeinkopplungsvorrichtung zum Einkoppeln von Energie in die implantierte Zentrifugalblutpumpenanordnung umfaßt.

Diese Aufgabe wird mit einer magnetischen Rotorlagerung der eingangs genannten Art erfindungsgemäß dadurch gelöst, daß der im Rotor angebrachte Permanentmagnet der Permanentmagnetanordnung, mittels deren der Rotor im Stator aufgehängt ist, bezüglich des zugeordneten und im Stator angebrachten Permanentmagneten dieser Permanentmagnetanordnung axial derart versetzt angeordnet ist, daß gleichsinnige Magnetpole dieser beiden Permanentmagneten einander zugwandt sind, wobei die Regelschaltung die Rotorposition in einer Lage stabilisiert, in welcher der absolute Energiebedarf der Elektromagnetanordung ohne äußere Kräfte minimalisiert ist.

Die letztere Funktion wird von einem Regelkreis mit festem Sollwert erfüllt.

Diese magnetische Rotorlagerung nach der Erfindung hat bei optimal radial zentrierender Kraftwirkung der Permanentmagnetanordnung auf den Rotor einen höchstmöglichen elektromechanischen Wirkungsgrad, da nur der geringstmögliche Anteil der zum Aufhängen und Stabilisieren des Rotors benötigten Magnetkräfte elektromagnetisch erzeugt wird, während der bei weitem größte Anteil der erforderlichen Magnetkräfte mittels Permanentmagneten aufgebracht wird und bei gegebener Größe des Magnetmaterials bzw. der Magnetanordnung, insbesondere bei gegebener Menge, bei gegebenem Volumen und bei gegebenem Gewicht des Magnetmaterials bzw. der Magnetanordnung, maximale Zentrierungskräfte in radialer Richtung vorhanden sind. Es sei in diesem Zusammenhang darauf hingewiesen, daß eine stabile Aufhängung des Rotors mittels Permanentmagneten allein gemäß dem Earnshaw-Theorem nicht möglich ist, wonach jedes nur mittels Permanentmagneten im Raum (drei Dimensionen) im Gleichgewicht gehaltene mechanische System instabil ist. Außerdem ist der absolute Energiebedarf der magnetischen Rotorlagerung nach der Erfindung so klein wie möglich, weil die Regelschaltung die Rotorposition in einer Lage stabilisiert, in welcher der absolute Energiebedarf der Elektromagnetanordnung ohne äußere Kräfte minimalisiert ist, während zur Minimalisierung bei Einwirkung äußerer Kräfte ein weiter unten angegebener übergeordneter Regler vorgesehen sein kann.

Die erfindungsgemäße magnetische Rotorlagerung ist daher mit besonderem Vorteil für alle die Fälle geeignet, in denen der Energiebedarf einer solchen magnetischen Rotorlagerung absolut und relativ gering sein sollte und außerdem die Temperaturerhöhung, welche die magnetische Rotorlagerung infolge der entstehenden Wärme erfährt, möglichst klein sein sollte. Infolgedessen ist die magnetische Rotorlagerung nach der Erfindung besonders vorteilhaft bei Blutpumpen, insbesondere Axial- und Radial-Zentrifugalblutpumpen anwendbar, vor allem in den Fällen, in denen derartige Blutpumpen implantiert werden sollen. Die verhältnismäßig geringe Wärmeentwicklung ermöglicht es, mit Sicherheit unterhalb der Temperatur von 42°C zu bleiben, bei welcher das Bluteiweiß zu koagulieren beginnt und die daher äußerst gefährlich für den menschlichen Blutkreislauf ist.

Eine bevorzugte Ausführungsform dieser magnetischen Rotorlagerung zeichnet sich erfindungsgemäß dadurch aus, daß der im Rotor angebrachte Permanentmagnet der Permanentmagnetanordnung, mittels deren der Rotor im Stator aufgehängt ist, bezüglich des zugeordneten und im Stator angebrachten Permanentmagneten dieser Permanentmagnetanordnung derart axial versetzt angeordnet ist, daß sich der im Rotor angebrachte Permanentmagnet gerade außerhalb der axialen Erstreckung des im Stator angebrachten Permanentmagneten befindet.

Eine andere bevorzugte Ausführungsform der magnetischen Rotorlagerung nach der Erfindung, die insoweit übereinstimmend mit der in EP-A-0 019 313 beschriebenen Magnetlagerung ausgebildet ist, als

(a) die den Rotor aufhängende Permanentmagnetanordnung einen konzentrisch zur Rotorachse angeordneten ortsfesten Ringpermanentmagneten und einen konzentrisch zu demselben auf der Rotorachse angeordneten Stabpermanentmagneten oder Ringpermanentmagneten umfaßt; und

(b) die Regelschaltung mit ihrem Istwert-Eingang an den die axiale Lage des Rotors ermittelnden Lagesensor bzw. dessen Lagesensorbetriebsschaltung angeschlossen ist;

zeichnet sich erfindungsgemäß dadurch aus, daß der Stabpermanentmagnet oder der Ringpermanentmagnet des Rotors axial innerhalb des Elektromagneten angeordnet ist.

Die Permanentmagnetanordnung der erfindungsgemäßen Rotorlagerung ist vorzugsweise wie folgt aufgebaut:

(1) Bei gegebenem Querschnitt der Permanentmagneten soll deren Länge so gewählt sein, daß sich der maximale äußere Streufluß ausbilden kann. Dazu muß die Länge des Permanentmagneten so gewählt werden, daß die vorhandene magnetische Spannung gerade ausreicht, um den Streufluß durch den magnetischen Widerstand im Streufeldbereich bzw. -volumen zu treiben.

(2) Als Permanentmagnetmaterial werden vorzugsweise Seltene Erden-Magnete, insbesondere Cobalt-Samarium-Magnete verwendet. Dieses Material wird in geringstem Maße unter dem Einfluß eines magnetischen Fremdfeldes geschwächt oder entmagnetisiert. Außerdem wird eine hohe magnetische Spannung bei geringster Magnetlänge erreicht. Schließlich ist die spezifische magnetische Energie des Materials bezogen auf das Gewicht, die größte aller Magnetmaterialien. Endlich ist die Homogenität der Magnetisierung des Materials relativ gut.

(3) Der äußere Durchmesser des Stab- oder Ringpermanentmagneten des Rotors muß in der Größenordnung des inneren Durchmessers des Ringpermanentmagneten des Stators liegen und etwas kleiner als dieser sein. Dadurch wird erreicht, daß z.B. bei einer radialen Verschiebung des Rotorpermanentmagneten gegenüber dem Statorpermanentmagneten eine, bezogen auf die Längeneinheit der Verschiebung, maximale Änderung der Gesamtmagnetenfeldenergie der Permanentmagnetanordnung stattfindet.

(4) Es wird nur extrem homogenes Magnetmaterial verwendet, weil sonst die Drehung des Rotors exzentrisch erfolgt, da die Symmetrieachse des Magnetfeldes andernfalls nicht mit der geometrischen Achse des Magneten übereinstimmt.

Bei der Permanentmagnetanordnung der erfindungsgemäßen Rotorlagerung bedarf lediglich der axiale Freiheitsgrad einer Stabilisierung, um die gesamte magnetische Rotorlagerung stabil zu machen, und diese Stabilisierung erfolgt durch die erwähnte, einen axialen Elektromagneten aufweisende Elektromagnetanordnung. Wesentliches Merkmal des erfindungsgemäß ausgebildeten gemischt permanentmagnetischen-elektromagnetischen Aufbaus ist die hierbei auftretende Wechselwirkung zwischen Permanentmagnetfeld und Elektromagnetfeld, wobei der axiale Freiheitsgrad dadurch mittels des Elektromagneten stabil gemacht wird, daß die Feldstärke dieses Elektromagneten durch einen Regelkreis geregelt wird, der eine die Position der Rotorachse in Axialrichtung ermittelnde Lagesensoreinrichtung umfaßt, durch welche ein die Ist-Position der Rotorachse in Axialrichtung angebendes Signal erzeugt wird, das im Regelkreis mit einem die Soll-Position der Rotorachse in Axialrichtung angebenden Sollwert verglichen wird, woraufhin die Regelschaltung dieses Regelkreises die elektrische Speisung des Elektromagneten so einregelt, daß die axiale position der Rotorachse in die Soll-Position gebracht und in dieser Soll-Position gehalten wird.

Eine besonders schnelle Rückstellung der Rotorachse in die Soll-Position dann, wenn die Rotorachse durch irgendwelche Kräfte aus dieser Soll-Position verschoben wird, wird dadurch erreicht, daß in Weiterbildung der Erfindung die Regelschaltung einen stromproportional regelnden Endverstärker aufweist Dadurch kann der Strom in der Erregungsspule des Elektromagneten innerhalb kürzester Zeit auf einen bestimmten Stromwert, der zur Rückführung der Rotorachse in deren axiale Soll-Position erforderlich ist, eingestellt werden. Eine Verzögerung des Feldaufbaus durch die Induktivität des Elektromagneten kann hierdurch eliminiert werden.

Die vorstehend angegebene Regelung mit fester axialer Soll-Position des Rotors ist hinsichtlich des Energieverbrauchs zwar für kurzzeitige, auf den Rotor einwirkende äußere Verstellkräfte optimal, da derartige kurzzeitige Kräfte definitionsgemäß nach verhältnismäßig sehr kurzer Zeit, nämlich nach einigen tausendstel, hundertstel oder zehntel Sekunden, wieder verschwinden. Wenn jedoch äußere axiale Verstellkräfte auf die Rotorachse einwirken, die langfristig, d.h. ab einigen zehntel Sekunden bis einige Stunden lang, wirken, dann kann sich ein wesentlich erhöhter Energieverbrauch für die Stabilisierung der Rotorachse in der vorerwähnten Soll-Position ergeben. Um diesen erhöhten Energieverbrauch zu vermindern, ist eine besonders bevorzugte Ausführungsform der magnetischen Rotorlagerung erfindungsgemäß derart ausgebildet, daß die Regelschaltung eine deren Sollwert beim langzeitigen Einwirken wesentlicher äußerer Kräfte direkt oder durch Veränderung des Istwerts des Lagesensors indirekt verschiebenden, den Energieverbrauch der magnetischen Rotorlagerung vermindernden, vorzugsweise minimalisierenden, übergeordneten Regler aufweist. Untersuchungen an praktischen Ausführungen der erfindungsgemäßen magnetischen Rotorlagerung haben gezeigt, daß der Leistungsbedarf dieser magnetischen Rotorlagerung bei Nichtvorhandensein wesentlicher äußerer Kräfte beispielsweise etwa 0,1 Watt für die Lageregelung der Rotorachse beträgt, und daß dieser Leistungsbedarf beim Einwirken wesentlicher äußerer Kräfte bis auf beispielsweise 10 bis 15 Watt ansteigen kann, also auf den 100- bis 150 fachen Betrag, wenn der vorerwähnte übergeordnete Regler nicht vorgesehen ist. Wesentliche äußere Kräfte sind insbesondere einseitig wirkende Beschleunigungskräfte, wie sie zum Beispiel in Kraftfahrzeug, Eisenbahn, Flugzeug o.dgl. beim Anfahren und Abbremsen auftreten, sowie die Erdbeschleunigung, sofern sie bezüglich der Axialrichtung des Rotors stark unsymmetrisch wird, was beispielsweise geschehen kann, wenn sich ein Patient, dem ein künstliches Herz mit einem Rotor implantiert ist, welcher mittels der magnetischen Rotorlagerung nach der Erfindung gelagert ist, beim Schlafen auf die Seite legt o.dgl. Wenn man bedenkt, daß bei einem solchen implantierten künstlichen Herzen die benötigte Leistung induktiv in den Körper des Menschen eingekoppelt werden muß, dann ist es ganz wesentlich, daß die zur Lagestabilisierung des Rotors benötigte Leistung anstatt, wie oben erwähnt, beispielsweise 10 bis 15 Watt nur etwa 0,1 Watt beträgt.

Im einzelnen umfaßt der übergeordnete Regler vorzugsweise folgendes:

(a) eine das Vorhandensein äußerer Kräfte ermittelnde Detektionseinrichtung;

(b) eine den Rotor aus seiner stabilisierten Mittenlage in eine außermittige Stabilisierungslage verstellende Verstelleinrichtung; und

(c) eine den Energiebedarf der Rotorlagerung in der stabilisierten Mittenlage mit deren Energiebedarf in der außermittigen Stabilisierungslage oder den Energiebedarf in zwei verschiedenen außermittigen Stabilisierungslagen vergleichende Vergleicheinrichtung.

Ein in dieser Art ausgebauter übergeordneter Regler arbeitet daher, wenn die Detektionseinrichtung wesentliche äußere Kräfte ermittelt, in der Weise, daß die Detektionseinrichtung die Verstelleinrichtung veranlaßt, den Rotor aus der bisherigen stabilisierten Mittenlage ein Stück weit in eine außermittige Stabilisierungslage zu verstellen, so daß permanentmagnetische Kräfte der Lagermagneten, die bei einer unsymmetrischen Lage des Rotors auftreten, den äußeren Kräften entgegenwirken.

Daraufhin erfolgt mittels der Vergleicheinrichtung ein Vergleich des vorherigen Energiebedarfs mit dem neuen Energiebedarf der magnetischen Rotorlagerung, und je nachdem, ob der neue Energiebedarf höher oder geringer als der vorhergehende Energiebedarf ist, wird die Verstelleinrichtung veranlaßt, den Rotor in einem nächsten Schritt erneut im Sinne einer Verminderung des Energiebedarfs der magnetischen Rotorlagerung zu verstellen, und dieses schrittweise oder kontinuierliche Verstellen erfolgt bevorzugt so lange, bis eine neue Stabilisierungslage der Rotorachse erreicht ist, bei welcher die magnetische Rotorlagerung bei der jeweiligen wesentlichen äußeren Kraft einen minimalen Energiebedarf hat.

Durch entsprechende Ansprechverzögerung kann erreicht werden, daß der übergeordnete Regler auf kurzzeitige äußere Kräfte, beispielsweise solche, die nur Bruchteile von Sekunden dauern, nicht anspricht.

Obwohl Detektionseinrichtungen der verschiedensten Art für einen solchen übergeordneten Regler geeignet sind, ist es zu bevorzugen, als Detektionseinrichtung eine Strommeßeinrichtung zum Messen des durch die Elektromagnetanordnung fließenden elektrischen Stromes zu verwenden, oder eine Strommeßeinrichtung zum Messen des gesamten elektrischen Stromes, welcher durch den den Lagesensor bzw. die Lagesensoren, die Regelschaltung und die Elektromagnetanordnung umfassenden Regelkreis fließt.

Eine bevorzugte Ausführungsform der Verstelleinrichtung ist eine Lagesensor-Sollwertveränderungseinrichtung, wobei dieser Sollwert, welcher der axialen Sollposition der Rotorachse entspricht, entweder direkt verändert werden kann, indem der in den Regelkreis eingegebene Sollwert entsprechend verändert wird, oder indirekt, indem der in die Regelschaltung eingegebene Sollwert konstant gehalten, jedoch zu dem vom jeweiligen Lagesensor abgegebenen Istwert eine zusätzliche Größe addiert oder von diesem Istwert eine zusätzliche Größe subtrahiert wird.

Schließlich umfaßt die Vergleicheinrichtung vorzugsweise eine Strommeßeinrichtung zum Messen des durch die Elektromagnetanordnung oder durch den gesamten Regelkreis fließenden elektrischen Stromes und eine die Strommeßwerte speichernde und vergleichende Sample- und Hold-Schaltung, u. U. ist dadür auch ein Verzögerungsglied brauchbar, solange der Wert lang genug gehalten wird.

Als Lagesensoren können im Prinzip die unterschiedlichsten, nach dem Stande der Technik bekannten Lagesensoren verwendet werden, solange die Lageinformation nur im Stator vorliegt und solange ein evtl. im Rotor vorliegender Sensorteil keine Energie bzw. keine elektrische Kontaktierung benötigt. Jedoch sind Magnetfeldsensoren im allgemeinen nicht brauchbar (Magnet im Rotor), weil solche Sensoren vom Magnetfeld der magnetischen Rotorlagerung beeinflußt werden können und sich dadurch ungünstige Rückwirkungen ergeben; kapazitive Sensoren zur Lageermittlung sind aus verschiedenen Gründen praktisch nicht brauchbar oder sehr problematisch, und zwar einmal deswegen, weil dadurch im Blut ein elektrisches Feld erzeugt wird und eine problematische Kontaktierung notwendig ist, wenn die Kondensatorplatten eines solchen Sensors zwischen Stator und Rotor vorgesehen sind, zum anderen deswegen, weil in den Fällen, in denen die beiden Kondensatorplatten des Sensors am Gehäuse vorgesehen werden, eine Änderung die Dielektrikums zwischen diesen Kondensatorplatten durch den Rotor erfolgt. Erfindungsgemäß werden als Lagesensoren bevorzugt Strahlungssensoren verwendet, insbesondere solche, die mit Lichtstrahlung oder Schallstrahlung arbeiten, vorzugsweise Infrarotsensoren oder Ultraschallsensoren.

Im einzelnen ist die magnetische Rotorlagerung nach der Erfindung hinsichtlich ihrer Lagesensoranordnung vorzugsweise so aufgebaut, daß der Lagesensor wenigstens eine von der Rotorwelle betätigte Impulsstrahlungsschranke mit einem impulsgesteuerten Strahlungssendeelement und einem Strahlungsempfangselement umfaßt.

Die Lagesensoreinrichtung, die bevorzugt zwei Lagesensoren und eine daran angekoppelte Lagesensorbetriebsschaltung aufweist, kann insbesondere folgendes umfassen:

(1) zwei Impulsstrahlungsschranken, von denen je eine mit je einem axialen Ende der Rotorwelle zusammenwirkt;

(2) eine die Ausgangssignale der Strahlungsempfangselemente der beiden Impulsstrahlungsschranken subtrahierende Substraktionseinrichtung;

(3) eine die Ausgangssignale der Substraktionseinrichtung integrierende Integrationseinrichtung;

(4) eine an den Ausgang der Integrationseinrichtung angeschlossene Sample- und Hold-Schaltung; und

(5) eine Ablaufsteuereinrichtung zur Steuerung des Schließ- Öffnungstakts eines ersten, zweiten und dritten Schalters, von denen der erste Schalter zwischen dem Ausgang der Substraktionseinrichtung und dem Eingang der Integrationseinrichtung vorgesehen ist, während der zweite Schalter ein die Rückstellung der Integrationseinrichtung bewirkender Schalter ist und der dritte Schalter zwischen dem Ausgang der Integrationseinrichtung und dem Eingang der Sample- und Hold-Schaltung angeordnet ist, wobei die Ablaufsteuereinrichtung die Schließzeiten der drei Schalter derart steuert, daß der erste Schalter immer dann geschlossen ist, wenn die Strahlungssendeelemente senden, während das Schließen des dritten Schalters jeweils nach Integration einer vorbestimmten Anzahl von Ausgangssignalen der Subtraktionseinrichtung erfolgt und der zweite Schalter jedesmal nach Öffnen des dritten Schalters geschlossen wird.

Wie sich aus den obigen Ausführungen über die bevorzugt verwendeten Sensoren bereits ergibt, sind die Impulsstrahlungsschranken vorzugsweise entweder Infrarotlichtschranken oder Ultraschallschranken. Der Vorteil der Infrarotsensoren bzw. -lichtschranken und der Ultraschallsensoren bzw. -schranken besteht insbesondere darin, daß beide Arten dieser Sensoren bzw. Schranken von den elektrischen oder magnetischen Eigenschaften der Umgebung entkoppelt sind, weil Infrarotlicht oder Ultraschallstrahlung praktisch nicht von diesen elektrischen oder magnetischen Eigenschaften beeinflußt werden. Abgesehen hiervon haben die bei der Anwendung von Infrarotlicht verwendeten LED-Dioden einen sehr geringen Energiebedarf und sind preiswert sowie klein.

In Verbindung mit der vorstehend beschriebenen magnetischen Rotorlagerung oder auch unabhängig hiervon kann als Antrieb des Rotors ein Asynchronmotor vorgesehen sein, der an einen Oszillator als Betriebsspannungsquelle angeschlossen ist, dessen Frequenz und Ausgangsspannungsamplitude last- und drehzahlabhängig derart veränderbar sind, daß das vom Asynchronmotor abgegebene Drehmoment bei der jeweiligen Drehzahl des Asynchronmotors mit dem Lastmoment korreliert, insbesondere das jeweilige Lastmoment des Rotors auf der Motorkennlinie des Asynchronmotors wenig unterhalb des Kippmoments und oberhalb der Kippdrehzahl liegt.

Es ist weiter ein elektronisch kommutierterter Gleichstromantrieb besonders vorteilhaft. Hierzu können insbesondere Permanentmagneten in Form von Magnetscheiben oder sonstigen magnetisierten Bereichen im Rotor, vorzugsweise um dessen äußeren Umfang herum bzw. in dessen äußerem Umfang, als alternierende Magnetpole vorgesehen sein, denen im Stator beidseitig oder einseitig eisenlose stromdurchflossene Drahtschleifen gegenüberstehen, die möglichst den gesamten magnetischen Fluß der vorgenannten Permanentmagneten des Rotors erfassen, wobei der elektrische Strom in den Drahtschleifen so kommutiert wird, daß eine Rotation des Rotors bewirkt wird.

Bei Verwendung des Rotors als Förderorgan in einer Blutpumpe, insbesondere einer Zentrifugalblutpumpe, kann eine Antriebsleistungssteuereinrichtung vorgesehen sein, die folgendes umfaßt:

(a) einen Drucksensor auf der Saugseite der Blutpumpe;

(b) einen mit seinem Eingang an den Drucksensor angeschlossenen und einer vorbestimmten Eingangsgröße eine bestimmte Ausgangsgröße zuordnenden und abgebenden Charakteristikspeicher; und

(c) eine die tatsächliche Blutpumpenleistung entsprechend der Ausgangsgröße des Charakteristikspeichers steuernde Leistungssteuereinrichtung, vorzugsweise einen in seiner Frequenz und Ausgangsspannung last- und drehzahlabhängig veränderbaren Oszillator, der an einen Asynchronmotor, welcher die Blutpumpe antreibt, als die Betriebsspannungsquelle dieses Asynchronmotors angeschlossen ist.

Obwohl der Antrieb eines magnetischen Rotors, wie er mit der Erfindung zur Verfügung gestellt wird, bevorzugt in der vorstehend genannten Weise erfolgt, kann der Antrieb aber alternativ auch auf irgendeine andere geeignete Weise ausgebildet sein, zum Beispiel ein Synchronmotor, ein elektronisch kommutierter permanentmagnetisch erregter Elektromotor o. dgl. sein. Und obwohl der vorstehend beschriebene Asynchronmotorantrieb bevorzugt bei einem Rotor angewandt wird, welcher eine erfindungsgemäß ausgebildete magnetische Rotorlagerung besitzt, kann dieser Asynchronmotorantrieb auch mit Vorteil bei anders gelagerten Rotoren verwendet werden, zum Beispiel bei solchen Rotoren, die ausschließlich mittels Elektromagneten magnetisch gelagert sind, oder bei Rotoren, die eine gemischt elektromagnetische-permanentmagnetische Rotorlagerung der Art haben, wie sie in der US-Patentschrift 3 938 913 und in der europäischen Offenlegungsschrift 0 060 569 beschrieben ist, oder bei irgendeiner anderen magnetischen oder mechanischen oder hydraulischen oder sonstigen Rotorlagerung.

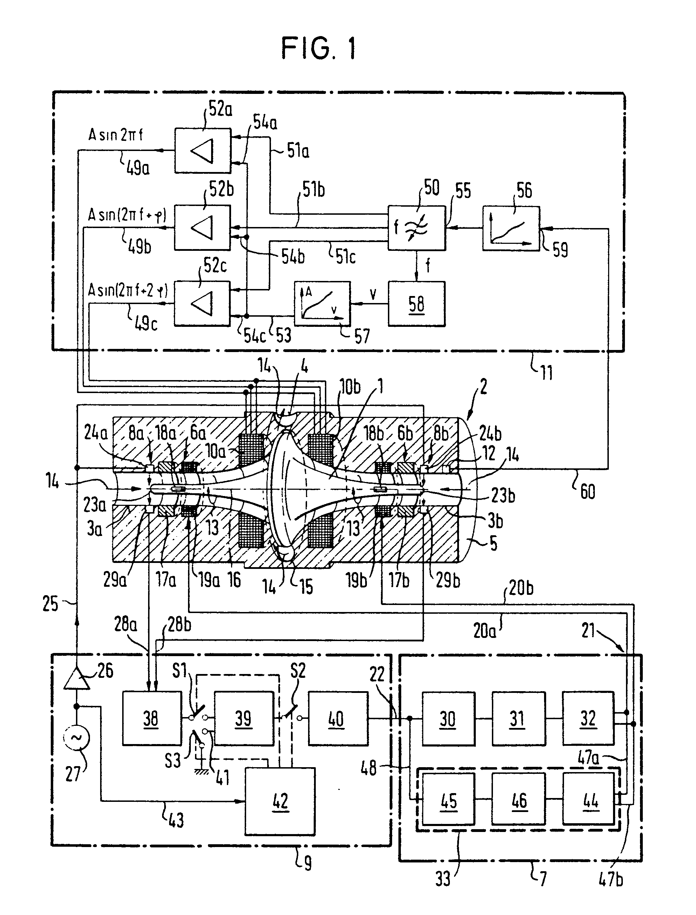

Die vorstehenden sowie weitere Vorteile und Merkmale der Erfindung seien nachfolgend unter Bezugnahme auf die Fig. 1 bis 5 der Zeichnung anhand einiger besonders bevorzugter Ausführungsformen der erfindungsgemäßen Rotorlagerung und anhand einer bevorzugten Ausführungsform eines Rotorantriebs nach der Erfindung näher erläutert; es zeigen.

Fig. 1 eine Gesamtdarstellung einer besonders bevorzugten Ausführungsform einer magnetischen Rotorlagerung nach der Erfindung und einen erfindungsgemäßen Rotorantrieb eines Rotors einer doppelflutigen Radial-Zentrifugalblutpumpe die per, spektivisch zusammen mit den Permanent- und den Elektromagneten, in denen der Rotor dieser Zentrifugalblutpumpe magnetisch berührungslos gelagert ist bzw. angetrieben wird, dargestellt ist, während die Regelschaltung der magnetischen Rotorlagerung und die Antriebsschaltung für den Rotor in Blockdarstellung veranschaulicht sind;

Fig. 2 eine Schnittansicht durch eine zur magnetischen Lagerung eines Rotors einer Blutpumpe nach Fig. 1 dienende Magnetanordnung mit gestrichelt eingezeichnetem Rotor;

Fig. 3 eine bevorzugte Ausführungsform einer portional regelnden Endstufe, die in der Regelschaltung der magnetischen Rotorlagerung nach Fig. 1 vorgesehen ist;

Fig. 4a und 4b je eine Kurvendarstellung, die den zeitlichen Verlauf der Spannung bzw. des Stroms zeigt, die an bzw. der Wicklung des Elektromagneten erzielbar ist, welcher an die in Fig. 3 gezeigte Endstufe angeschlossen ist;

Fig. 5 eine Kurvendarstellung, die ein Beispiel der optimalen Wahl der Betriebsparameter des Asynchronmotors veranschaulicht, der in Fig. 1 als Rotorantrieb verwendet wird, und

Fig. 6 eine Ausführungsform einer Axial-Zentrifugalblutpumpe nach der Erfindung.

Die magnetische Rotorlagerung und der Rotorantrieb, die in Fig. 1 dargestellt sind, dienen hier zum Lagern und Antrieb des Rotors 1 einer doppelflutigen Radial-Zentrifugalblutpumpe 2, welche zwei Blutzuflußkanäle 3a und 3b und einen Blutauslaß 4 hat, die in einem als Gehäuse der Zentrifugalblutpumpe 2 dienenden Stator 5 ausgebildet sind, in welchem der Rotor 1 magnetisch berührungslos gelagert ist.

Die magnetische Rotorlagerung umfaßt eine erste und zweite mit 6a bzw. 6b bezeichnete Permanent- und Elektromagnetanordnung in je einem der beiden axialen Endbereiche des Rotors 1 sowie eine Regelschaltung 7 zum Betrieb des Elektromagneten der Permanent- und Elektromagnetanordnungen 6a und 6b. Außerdem umfaßt die magnetische Rotorlagerung zwei, jeweils als Impulsstrahlungsschranken 8a und 8b ausgebildete Lagesensoren für die Ermittlung der axialen Position des Rotors 1 und eine Lagesensorbetriebsschaltung 9, die Eingangssignale von den Impulsstrahlungsschranken 8a und 8b erhält und ein entsprechendes Ausgangssignal an die Regelschaltung 7 abgibt.

Der Rotorantrieb umfaßt zwei Antriebselektromagneten 10a und 10b, die zusammen mit dem metallischen Körper des Rotors 1 einen Asynchronmotor bilden, und eine Antriebsleistungssteuereinrichtung 11 mit einem auf der Saugseite der Zentrifugalblutpumpe 2, vorliegend im Blutzuflußkanal 3b, angeordneten Drucksensor 12. Die Drehung des Rotors 1 ist durch Pfeile 13 angedeutet, während der Blutfluß durch Pfeile 14 dargestellt ist.

Der Rotor 1 samt seiner Achse, der beispielsweise aus poliertem Aluminium bestehen kann, ist innen hohl, so daß seine Gesamtdichte genau derjenigen des ihn umgebenden Blutes angepaßt ist. Durch die beiden Blutzuflußkanäle 3a und 3b wird der Blutstrom von beiden Seiten an den scheibenförmigen Rotor 1 herangeführt und von diesem zentrifugal über einen Blutringkanal 15 zentrifugal aus dem als Gehäuse dienenden Stator 5 getrieben, so daß infolgedessen keine Asymmetrischen Kräfte auf den Rotor 1 durch dessen Betrieb entstehen.

Der Energiebedarf des Rotorantriebs beträgt in einer beispielsweisen Ausführungsform etwa 8-10 Watt bei einem konstanten Blutdruck von 0,133 bar und ist damit wesentlich niedriger als derjenige von pulsierend arbeitenden Blutpumpen. Der Energiebedarf der magnetischen Rotorlagerung beträgt in dieser beispielsweisen Ausführungsform nur etwa 0,5 Watt. Aufgrund dieses geringen Energiebedarfs können die Batterie- oder Akkumulatorensätze zur Energieversorgung verhältnismäßig klein und leicht sein und implantiert werden und brauchen erst nach einem oder zwei Tagen erneuert oder z.B. nachts neu aufgeladen zu werden.

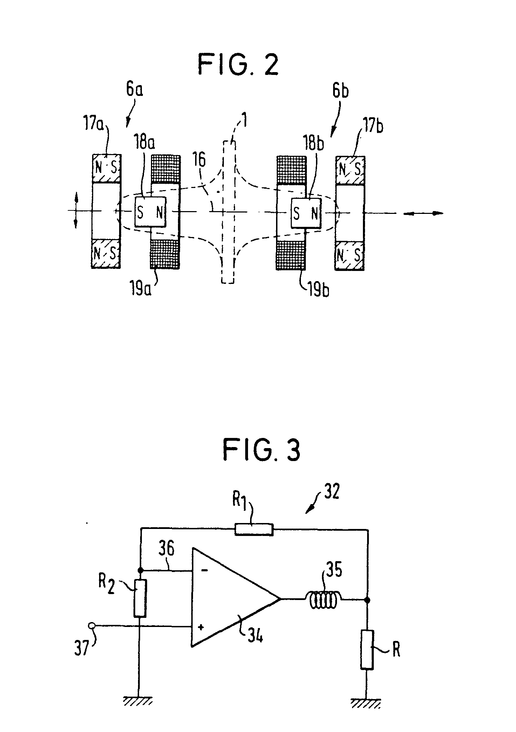

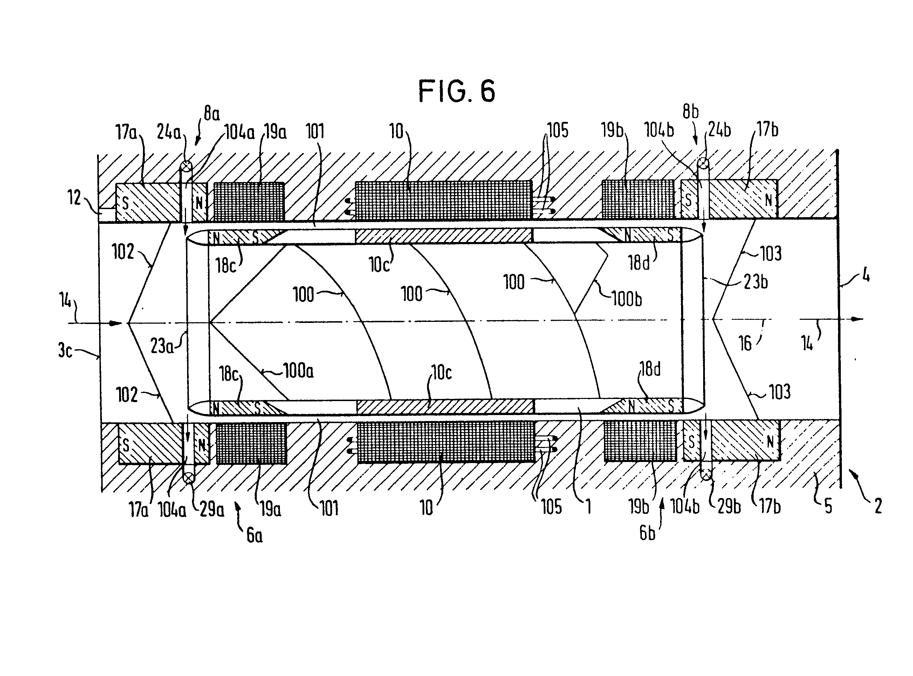

Im einzelnen sind die Permanent- und Elektromagnetanordnungen 6a und 6b der magnetischen Rotorlagerungen, die in Fig. 2 in gegenüber Fig. 1 vergrößertem Maßstab dargestellt sind, so ausgebildet, daß der Rotor 1 im Bereich jedes seiner beiden axialen Enden mittels einer Permanentmagnetanordnung bis auf einen einzigen geometrischen Verstellfreiheitsgrad, der sich vorliegend in Axialrichtung des Rotors 1 erstreckt, stabil berührungslos im Stator 5 aufgehängt ist. Diese den Rotor 1 aufhängende Permanentmagnetanordnung umfaßt einen konzentrisch zur Rotorachse 16 angeordneten Ringpermanentmagneten 17a bzw. 17b, der ortsfest im Stator 5 vorgesehen ist, und einen konzentrisch zum Ringpermanentmagneten 17a, 17b auf der Rotorachse 16 und innerhalb des Rotors 1 angeordneten Stabpermanentmagneten 18a bzw. 18b (Fig. 1 und 2) oder Ringpermanentmagneten 18c bzw. 18d (Fig. 6).

Im einzelnen ist der Ringpermanentmagnet 17a bzw. 17b in Axialrichtung des Rotors 1 magnetisiert, wie in Fig 2 und 6 durch die Angabe der Nordpole N und Südpole S angedeutet ist. Weiter ist der Stabpermanentmagnet 18a bzw. 18b bzw. der Ringpermanentmagnet 18e bzw. 18d des Rotors bezüglich des zugeordneten Ringpermanentmagneten 17a bzw. 17b des Stators axial derart versetzt angeordnet, daß er sich gerade außerhalb des Ringpermanentmagneten 17a bzw. 17b des Stators befindet bzw. diesen eben noch berührt. Auf diese Weise ergibt sich eine optimale radial zentrierende Kraftwirkung der jeweils aus einem Ringpermanentmagneten 17a bzw. 17b im Stator und einem Stabpermanentmagneten 18a bzw. 18b bzw. Ringpermanentmagneten 18c bzw. 18d im Rotor bestehenden Permanentmagnetanordnung. Auch die Stabpermanentmagnete 18a und 18b bzw. die Ringpermanentmagnete 18c und 18d sind in Axialrichtung des Rotors magnetisiert (siehe die Polangaben in Fig. 2 und 6), und sie rotieren zusammen mit dem Rotor 1 um die Rotorachse 16 oder können frei drehbar im Rotor 1 gelagert sein, so daß sie sich dann nicht mit demselben mitdrehen.

Außerdem weist jede der beiden Permanent- und Elektromagnetanordnungen 6a und 6b je einen ringförmigen Elektromagneten 19a bzw. 19b auf, innerhalb dessen der Stabpermanentmagnet oder der Ringpermanentmagnet des Rotors angeordnet ist. Die Elektromagnete 19a und 19b sind konzentrisch zur Rotorachse 16 ortsfest im Stator 5 vorgesehen. Infolgedessen befindet sich der Elektromagnet 19a bzw. 19b in magnetischer Wechselwirkung mit dem zugeordneten zylinderförmigen Stabpermanentmagneten 18a bzw. 18b oder dem Ringpermanentmagneten 18c bzw. 18d des Rotors 5, wobei sich der Stabpermanentmagnet 18a bzw. 18b bzw. der Ringpermanentmagnet 18c bzw. 18d des Rotors 5 seinerseits in magnetischer Wechselwirkung mit dem zugeordneten Ringpermanentmagneten 17a bzw. 17b des Stators befindet. Durch die Elektromagneten 19a und 19b in Wechselwirkung mit den Permanentmagneten 18a und 18b bzw. 18c und 18d wird die axiale Lage des Rotors 1 zentriert und stabilisiert. In Symmetrie und Mittellage kompensieren sich die axialen Permanentmagnet-Kräfte gerade, so daß labiles Gleichgewicht entsteht.

Um die Zentrierung und Stabilisierung zu bewirken, wird das Magnetfeld der beiden Elektromagneten 19a und 19b, die über entsprechende Leitungen 20a bzw. 20b an den Ausgang 21 der Regelschaltung 7 angeschlossen sind, laufend geregelt.

In Fig. 6 ist eine Ausführungsform einer Axial-Zentrifugalblutpumpe dargestellt, in der für gleichartige Teile wie in Fig. 1 und 2 die gleichen Bezugszeichen verwendet worden sind, wobei nachstehend nur die demgegenüber unterschiedlichen Merkmale beschrieben sind, im übrigen aber auf die Beschreibung der Figuren 1 und 2 verwiesen wird.

Im Gegensatz zu der oben erläuterten Radial-Zentrifugalpumpe ist der Rotor 1 in Fig. 6 rohrförmig ausgebildet, und am inneren Rohrumfang desselben sind Flügel- bzw. Schraubenprofile 100 zum Fördern des Bluts durch das Rohrinnere vorgesehen, die am Bluteintritt bei 100a und am Blutaustritt bei 100b zugespitzt verlaufen. Anstelle der in Fig. 1 und 2 vorgesehenen Stabpermanentmagnete 18a und 18b sind hier im Rohrmantel des Rotors 1 an beiden axialen Enden die bereits erläuterten Ringpermanentmagnete 18c und 18d vorgesehen, wobei die gleiche Polarisationsart (einander entgegengesetzte Pole gegenüberliegend) wie in Fig. 1 und 2 angewandt ist (siehe die Polbezeichnungen N und S in Fig. 6).

Im Zwischenraum l01 zwischen dem Außenumfang des Rotors 1 und dem Innenumfang des Stators 5 sollte vorzugsweise eine Rückströmung des Bluts entgegengesetzt zu den Richtungen der Pfeile 14 verhindert oder vermindert werden. Das kann durch Förderflügel (nicht dargestellt) geschehen, die auf dem Außenumfang des Rotors 1 oder in Öffnungen vorgesehen sind, welche zwischen dem Ringpermanentmagneten 18c und/oder 18d und dem Kurzschlußring 10c des Rotors 1 ausgebildet sind und durch den Rohrmantel des Rotors 1 hindurchgehen, oder dadurch, daß die zuletzt erwähnten Öffnungen als in Radial- und Axialrichtung schraubenförmig verlaufende Förderkanäle ausgebildet sind, die das Blut in Richtung der Pfeile 14 im Zwischenraum 101 fördern.

Außerdem können im Blutzuflußkanal 3c vor dem Rotor 1 und/oder im Blutauslaß 4 hinter dem Rotor 1 feststehende Umlenkschaufeln 102, 103 zum Erreichen eines zufriedenstellenden Wirkungsgrades vorgesehen sein.

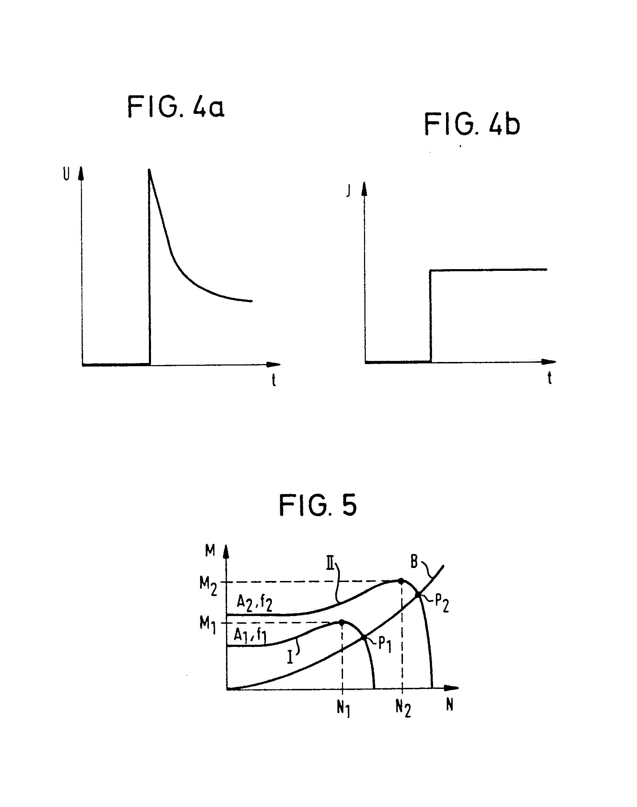

Zum Antrieb der Axial-Zentrifugalblutpumpe der Fig. 6 ist anstelle der beiden Elektromagneten 10a und 10b der Fig. 1 ein einziger Elektromagnet 10 im Stator 5 angeordnet, der mit dem Kurzschlußring 10c des Rotors 1 in der gleichen Weise wie anhand der Fig. 1 und 5 erläutert, als Asynchronmotor zusammen wirkt. Bei 105 sind die Erregerwicklungen des Elektromagneten 10 angedeutet.

Die Impulsstrahlungsschranken 8a und 8b wirken durch Bohrungen 104a und 104b im Ringpermanentmagneten 17a bzw. 17b mit den zugespitzt verlaufenden axialen Enden 23a und 23b des Rotors 1 zusammen.

Es sei nun die stabilisierende und zentrierende Regelung der axialen Rotorposition in den Zentrifugalblutpumpen nach Fig. 1 und 6 näher erläutert.

Zum Zwecke dieser Regelung ist der Istwert-Eingang 22 der Regelschaltung 7 an den Ausgang der Lagesensorbetriebsschaltung 9 angeschlossen, an dem ein Signal erhalten wird, welches die tatsächliche Position des Rotors 1 repräsentiert, wie sie mittels der Impulsstrahlungsschranken 8a und 8b in Zusammenwirken mit den axialen Enden 23a und 23b des Rotors 1 abgefühlt wird.

Zu Ihrem Betrieb sind die Strahlungssendeelemente 24a und 24b, beispielsweise je eine im Infrarotbereich arbeitende LED-Diode oder je ein Ultraschallsender, über eine Leitung 25 an den Ausgang eines Verstärkers 26 angekoppelt, dessen Eingang mit dem Ausgang eines Oszillators 27 verbunden ist. Der Verstärker 26 und der Oszillator 27 gehören zu der Lagesensorbetriebsschaltung 9, deren Signaleingänge 28a und 28b an je eines der Strahlungsempfangselemente 29a bzw. 29b der Impulsstrahlungsschranken 8a bzw. 8b angeschlossen sind. Diese Strahlungsempfangselemente 29a und 29b sind vorzugsweise ein Infrarotsensor oder ein Ultraschallsensor.

Die Regelschaltung 7, die ihren Istwert von der Lagesensorbetriebsschaltung 9 oder im einfachsten Fall von einem die axiale Position des Rotors 1 ermittelnden Lagesensor erhält, regelt die elektrische Stromstärke in den Elektromagneten 19a und 19b so, daß der am Istwert-Eingang 22 erhaltene Istwert schließlich mit einem vorgegebenen Sollwert für die axiale Position des Rotors 1 übereinstimmt.

Zu diesem Zweck umfaßt die Regelschaltung 7 z.B. einen unmittelbar an den Istwert-Eingang 22 angeschlossenen Vorverstärker 30, dessen Ausgang an einen nachfolgenden Regelverstärker 31 angeschlossen ist, der seinerseits einen stromproportional regelnden Endverstärker 32 aussteuert. Außerdem weist die Regelschaltung 7 einen hierarchischen bzw. übergeordneten Regler 33 auf, der weiter unten näher erläutert ist.

Der Regelverstärker 31 ist vorzugsweise ein PDT-Regelverstärker, d.h. ein Proportional-Differential-Regelverstärker mit Zeitglied. Die Vorteile dieses Regelverstärkers bestehen insbesondere darin, daß daß Systemrauschen unterdrückt und die Regelstabilität verbessert wird.

Der Endverstärker 32 wandelt eine gegebene Eingangsspannung in einen proportionalen Ausgangsstrom um, und zwar unabhängig von den elektrischen Lastverhältnissen. Auf diese Weise lassen sich trotz der relativ großen Induktivität der geregelten Elektromagnete 19a und 19b nahezu augenblickliche, d.h. z.B. treppenförmige Stromanstiege erzielen, indem die Spannung, wie in Fig. 4a gezeigt ist, zunächst wesentlich stärker erhöht wird, als es dem gewünschten Stromanstieg entspricht, und dann verhältnismäßig schnell auf einen, dem gewünschten Strom tatsächlich entsprechenden Spannungswert abgesenkt wird.

Ein Beispiel eines stromproportional regelnden Endverstärkers 32 ist in Fig. 3 dargestellt. Dieser Endverstärker 32 umfaßt einen Operationsverstärker 34, der über die als Last vorgesehene Magnetfeldspule 35 des Elektromagneten 19a bzw. 19b und einen niederomigen Widerstand R an Masse gelegt ist. Die am Widerstand R abfallende Spannung, die proportional dem Strom durch die Magnetfeldspule 35 ist, wird über einen aus den Widerständen $R_1$ und $R_2$ gebildeten Spannungsteiler auf den negativen Eingang 36 des Operationsverstärkers 34, der auch als Gegenkopplungseingang bezeichnet werden kann, zurückgeführt. Der positive Eingang 37 des Operationsverstärkers 34 ist an den Ausgang des Regelverstärkers 31 angekoppelt.

Es seien nun der Aufbau und die Betriebsweise der Impulsstrahlungsschranken 8a und 8b und der Lagesensorbetriebsschaltung 9 erläutert:

Wie bereits erwähnt, wirkt die Impulsstrahlungsschranke 8a mit dem einen axialen Ende 23a des Rotors 1 zusammen, so daß je nach der axialen Position des Rotors Strahlung von dem Strahlungssendeelement 24a, wie durch die beiden kleinen Pfeile angedeutet, zu dem zugeordneten Strahlungsempfangselement 29a gelangen kann oder durch das axiale Ende 23a des Rotors 1 daran gehindert wird, auf das Strahlungsempfangselement 29a zu fallen. In entsprechender Weise wirken das Strahlungssendeelement 24b und das Strahlungsempfangselement 29b mit dem anderen axialen Ende 23b des Rotors zusammen. Im vorliegenden Fall sind als Strahlungssendeelement Infrarotstrahlungssendeelemente und als Strahlungsempfangselemente Infrarotstrahlungsempfangselemente vorgesehen.

Der Oszillator 27, der beispielsweise auch allgemein ein Impulsgenerator sein kann, hat bevorzugt eine Frequenz, die im Bereich von 10 bis 100 kHz liegt, und zwar besonders bevorzugt 40 kHz beträgt. Die Infrarotstrahlungssendeelemente sind so gewählt, daß sie Infrarotstrahlung in einem Wellenlängenbereich erzeugen, in dem Blut eine geringe Absorptionsfähigkeit hat, d.h. bei etwa 1 nm.

Hinsichtlich des obigen Frequenzbereichs des Oszillators 27 sei darauf hingewiesen, daß das System zu langsam reagieren würde, wenn die Impulsfrequenz wesentlich niedriger als 10 kHz gewählt werden würde, und zwar aufgrund der Signalverarbeitung in dem weiter unten erläuterten Integrator; und wenn andererseits die Impulsfrequenz des Oszillators 27 wesentlich höher als 100 kHz gewählt wird, dann ist der elektrooptische Wirkungsgrad, der als Infrarotstrahlungssendeelemente verwendeten lichtemittierenden Dioden ungünstig und die Auswertung durch Hochfrequenz-Effekte erschwert.

Die Infrarotstrahlungsempfangselemente sind vorzugsweise Photodioden oder -transistoren, deren maximale spektrale Empfindlichkeit im gleichen Bereich wie die maximale Emmissionsfähigkeit der Infrarotstrahlungssendeelemente liegt.

Der Teil der Lagesensorbetriebsschaltung 9, mittels dessen die Ausgangssignale der Strahlungsempfangselemente 29a und 29b zu dem an den Eingang 22 der Regelschaltung 7 abzugebenden Ist wert verarbeitet werden, umfaßt eine Subtraktionseinrichtung 38, welche die Signale von den beiden Strahlungsempfangselementen 29a und 29b voneinander subtrahiert, sowie eine nachgeschaltete Integrationseinrichtung 39, der eine Samp- und Hold-Schaltung nachgeschaltet ist. Außerdem ist ein erster Schalter S1 zwischen dem Ausgang der Subtraktionseinrichtung 38 und dem Integrationseingang der Integrationseinrichtung 39 vorgesehen; ein zweiter Schalter S2 ist zwischen dem Rückstellanschluß 41 der Integrationseinrichtung 39 und Masse vorgesehen und dient dazu, die Integrationseinrichtung 39 jeweils zurückzustellen; und schließlich ist ein dritter Schalter S3 zwischen dem Ausgang der Integrationseinrichtung 39 und dem Eingang der Abtast- und Halteschaltung 40 angeordnet, deren Ausgang an den Istwert-Eingang 22 der Regelschaltung angekoppelt ist. Zur Steuerung des Schließens und Öffnens der Schalter S1, S2 und S3 ist eine Ablaufsteuereinrichtung 42 in der Lagesensorbetriebsschaltung 9 vorgesehen, die Eingangssignale von dem Oszillator 27 erhält, da die Schalter S1, S2 und S3 in einer weiter unten näher erläuterten Weise synchron mit den Signalen geöffnet und geschlossen werden, welche an den Signaleingängen 28a und 28b eintreffen.

Damit die Lagesensorbetriebsschaltung 9 nicht einfach die elektrischen Impulse, welche aufgrund der Strahlungsimpulse von den Strahlungsempfangselementen 29a und/oder 29b abgegeben werden, mit hoher Zeitkonstante integriert, was eine Ansprechverzögerung der gesamten Regelung der Elektromagneten 19a und 19b bedeuten und damit unter Umständen zu Eigenschwingungen führen würde oder bei Einwirkung schneller äußerer Kräfte die Lage des Rotors nicht mehr garantiert, sind der Aufbau und die Betriebsweise der Lagesensorbetriebsschaltung 9 im einzelnen wie folgt:

Zunächst ist zwischen die Strahlungsempfangselemente 29a und 29b, die im Falle der Verwendung von Infrarotstrahlung z. B. Phototransistoren oder -dioden oder sonstige Photoempfänger sind, und die gleichzeitig als Vorverstärker arbeitende Integrationseinrichtung 39, die bereits erwähnte Subtraktionseinrichtung 38 geschaltet. Diese Subtraktionseinrichtung 38 subtrahiert die Ausgangssignale der Strahlungsempfangselemente 29a und 29b, was insbesondere folgenden Zweck und folgende Vorteile hat:

(a) Eine Störung durch Fremdstrahlung wird vermindert, und zwar insbesondere deswegen, weil eventuelle Fremdstrahlung, die von außen her kommt, etwa in gleicher oder ähnlicher Stärke an beiden Strahlungsempfangselementen 29a und 29b wirksam wird und sich bei der Subtraktion der Signale dieser Strahlungsempfangselemente der Anteil der Fremdstrahlung vollständig oder doch zu einem wesentlichen Teil gegenseitig aufhebt. Außerdem ist eine Fremdstrahlung, die genau mit 40 kHz und phasenrichtig das Sendesignal stört, sehr unwahrscheinlich.

(b) Der Arbeitsbereich der gesamten Sensoranordnung wird, was die Ermittlung der axialen Position des Rotors 1 anbetrifft, erweitert, und zwar in Verbindung mit der Anordnung der Impulsstrahlungsschranken 8a und 8b derart, daß sich diese Impulsstrahlungsschranken nur in einem kleinen Arbeitsbereich überlappen, d.h. daß der Übergangsbereich zwischen vollständigem Schließzustand und vollständigem Öffnungszustand dieser beiden Impulsstrahlungsschranken 8a und 8b nicht vollständig zusammenfällt, sondern sich diese Übergangsbereiche nur in einem, bezogen auf den jeweiligen gesamten Übergangsbereich kleinen Teil überlappen, was bedeutet, daß sich beide Impulsstrahlungsschranken 8a und 8b nur über eine kleine Länge ihres Übergangsbereichs hinweg gleichzeitig in diesem, ihrem Übergangsbereich befindet.

(c) Durch die Subtraktion der Ausgangssignale der beiden Strahlungsempfangselemente 29a und 29b wird die Kennlinie der Lagesensoranordnung, also der gesamten Anordnung aus den Pulsstrahlungsschranken 8a und 8b und der Lagesensorbetriebsschaltung 9 in dem vorstehend definierten überlappenden Arbeitsbereich linearisiert.

Die Integrationseinrichtung 39 arbeitet so, daß sie die am Ausgang der Subtraktionseinrichtung 38 erhaltenen Differenzimpulse, die durch die Subtraktion der pulsförmigen Ausgangssignale der Strahlungsempfangselemente 29a und 29b erhalten worden sind, periodisch nur über jeweils wenige einzelne Differenzimpulse, beispielsweise vier Differenzimpulse integriert und dann an ihrem Ausgang über den Schalter S3 diesen Integrationswert an die Abtast- und Halteschaltung 40 weitergibt. Die Integration kann eine von der Breite der Differenzimpulse unabhängige Amplitudenintegration sein; und die Integrationszeitkonstante kann sehr hoch gewählt werden, d.h. sie kann gegen Unendlich gehen.

Durch diese Art der Weiterverarbeitung der Differenzimpulse in der Integrationseinrichtung 39 werden insbesondere folgende Vorteile erzielt:

(1) Die gesamte Lagesensoranordnung reagiert trotz sehr großer Integrationszeitkonstanten der Integrationseinrichtung 39 praktisch sofort, d.h. nach wenigen einzelnen Differenzimpulsen, z.B. nach vier Differenzimpulsen, auf eine Amplitudenänderung, so daß die Lagesensoranordnung sehr schnell auf axiale Lageveränderungen des Rotors 1 anspricht, da gleichzeitig die Impulsfolgefrequenz, wie oben erwähnt, im Bereich zwischen 10 bis 100 kHz, bevorzugt bei 40 kHz liegt. Bei der vorgenannten bevorzugten Impulsfolgefrequenz beträgt daher die Ansprechzeit etwa 10 msec. Diese Ansprechzeit kann je nach Bedarf durch Veränderung der Impulsfolgefrequenz und/oder der Zahl der periodisch integrierten einzelnen Differenzimpulse je nach den praktischen Bedürfnissen vergrößert oder verkleinert und damit optimal eingestellt werden.

(2) Da sich normalerweise, allein schon bedingt durch die Trägheit des Rotors 1 innerhalb der vorerwähnten kurzen Ansprechzeit der gesamten Lagesensoranordnung keine relativ großen Änderungen der axialen Position des Rotors 1 ergeben, efolgt aufgrund der angewandten Integrationsweise die Veränderung des am Istwert-Eingang 22 auftretenden Istwerts, der praktisch gleich dem integrierten Wert ist, und damit die Einregelung der Elektromagnete 19a und 19b durch die Regelschaltung 7 auf einen neuen Wert der integrierten Amplituden der Differenzimpulse nicht abrupt sondern allmählich, so daß keine Istwert-Sprünge stattfinden, durch die Eigenschwingungen angeregt werden könnten.

In der Sample- und Hold-Schaltung 40 wird der am Ausgang der Integrationseinrichtung 39 erhaltene Integrationswert jeweils solange festgehalten und als Istwert auf den Istwert-Eingang 22 gegeben, bis der Sample- und Hold-Schaltung 40 über den Schalter S3 ein neuer Integrationswert von der Integrationseinrichtung 39 zugeführt wird.

Die Ablaufsteuereinrichtung 42 schließt und öffnet die Schalter S1, S2 und S3 in der nachfolgend angegebenen Weise, wozu sie ihre Taktfrequenz über eine Leitung 43 von dem Impulsgenerator bzw. Oszillator 27 erhält:

(a) Der erste Schalter S1 ist imer dann geschlossen, wenn die Strahlungssendeelemente 24a und 24b eingeschaltet sind und damit das Strahlungsempfangselement 29a und/oder das Strahlungsempfangselement 29b ein Ausgangssignal abgibt, also ein Differenzimpuls am Ausgang der Subtraktionseinrichtung 38 abgegeben wird. In der Zwischenzeit zwischen dem Auftreten von aufeinanderfolgenden Differenzimpulsen ist dagegen der Schalter S1 geöffnet. Das bedeutet, daß die Differenzimpulse der Subtraktionseinrichtung synchron zum Impulsbetrieb der Impulsstrahlungsschranken 8a und 8b an die Integrationseinrichtung 39 weitergegeben werden. Auf diese Weise wird der Einfluß von etwaiger Störstrahlung wesentlich vermindert.

(b) Der dritte Schalter S3 wird nach jeweils einer vorbestimmten Anzahl von Differenzimpulsen, beispielsweise nach jeweils vier Differenzimpulsen geschlossen, so daß jeder der periodisch durch die Integrationseinrichtung 39 erhaltenen Integrationswerte jeweils an die Sample- und Hold-Schaltung 40 weitergegeben und damit als Istwert für die Regelschaltung verfügbar gemacht wird.

(c) Nachdem der jeweilige Integrationswert durch Schließen des Schalters S3 an die Sample- und Hold-Schaltung 40 weitergegeben und der Schalter S3 wieder geöffnet worden ist, wird der zweite Schalter S2 geschlossen, um den alten Integrationswert in der Integrationseinrichtung 39 zu löschen.

(d) Der zweite Schalter S2 und der dritte Schalter S3 werden vorzugsweise jeweils nur innerhalb einer Öffnungsperiode des ersten Schalters Sl geschlossen; jedoch können die Schalter S2 und S3 auch längere Zeit geschlossen sein, sie dürfen jedoch niemals gleichzeitig geschlossen sein, sondern der Schalter S3 muß immer vor dem Schalter S2 geschlossen werden, und der Schalter S2 darf immer erst nach dem Öffnen des Schalters S3 geschlossen werden, damit nicht ein Null-Integrationswert an die Sample- und Hold-Schaltung 40 abgegeben und damit ein völlig falscher und zudem sprunghaft geänderter Istwert am Istwert-Eingang 22 erhalten wird. Solange unter Einhaltung dieser Bedingungen der Schalter S2 geschlossen ist, erfolgt einfach keine weitere Integration mehr, so daß durch eine längere Schließperiode des Schalters S2 auch die Ansprechzeit der gesamten Lagesensoranordnung auf axiale Positionsänderungen des Rotors 1 vergrößert werden kann. Wird der Schalter S3 unter Einhaltung der obigen Bedingungen länger geschlossen gehalten, so bedeutet das, daß dadurch die Anzahl der periodisch amplitudenintegrierten Differenzimpulse erhöht, also ebenfalls die Ansprechzeit der gesamten Lagesensoranordnung vergrößert wird.

Die Ablaufsteuereinrichtung 42 ist bevorzugt eine Digitalsteuereinrichtung, insbesondere schon deswegen, weil sie zur Betätigung der Schalter S1, S2 und S3 dient, die jeweils nur zwei Zustände, nämlich einen Öffnungs- oder Schließzustand haben.

Das allgemeine Prinzip, welches der vorstehend beschriebenen Lagesensorbetriebsschaltung 9 zugrundeliegt, besteht infolgedessen darin, daß die zur Ermittlung der axialen Position des Rotors 1 dienenden Lagesensoren, vorliegend die Impulsstrahlungsschranken 8a und 8b, impulsmäßig betrieben werden, daß durch Differenzbildung der Lagesensorsignale Störungen ausgeschaltet werden, daß die Integration der Differenzimpulse intermittierend über nur jeweils wenige Einzel-Differenzimpulse erfolgt, daß die zu integrierenden Differenzimpulse der Integrationseinrichtung synchron zum Auftreten der Strahlungsimpulse in den Lagesensoren zugeführt werden, und daß der intermittierend ermittelte Integrationswert auch in den Zwischenzeiten zwischen zwei Integrationsintervallen am Ausgang der Lagesensorbetriebsschaltung 9 und damit am Istwert-Eingang 22 der Regelschaltung 7 gehalten wird.

Ein etwaiger Einfluß von Störstrahlung auf die Lagesensoren und damit auf den Istwert der Regelschaltung wird insgesamt vor allem durch folgende Maßnahmen ausgeschaltet:

(1) Durch paarung von schmalbandigen Strahlungssendeelementen 24a und 24b mit schmalbandigen Strahlungsempfangselementen 29a und 29b, beispielsweise durch die Paarung von schmalbandigen Infrarot-Strahlungssendeelementen und Infrarot-Strahlungsempfangselementen, deren hauptsächlicher Strahlungssende- bzw. -empfangsbereich bei Infrarotstrahlung bei einer Wellenlänge von ca. 1 nm liegt;

(2) durch eine, vorstehend noch nicht erwähnte, kapazitive Kopplung mit niedriger Zeitkonstanten zwischen den Strahlungsempfangselementen 29a und 29b einerseits und der Subtraktionseinrichtung 38 andererseits, d.h. also durch ein Differenzierglied, das nur schnelle Spannungsänderungen durchläßt;

(3) durch die Subtraktionseinrichtung 38, wie oben näher erläutert;

(4) durch synchronisierte Übernahme der Differenzimpulse von der Subtraktionseinrichtung 38 in die Integrationseinrichtung mittels entsprechender Ablaufsteuerung, wie oben erläutert; und

(5) durch periodische Integration von jeweils nur mehreren einzelnen Differenzimpulsen, beispielsweise von vier Differenzimpulsen; hierdurch wird erreicht, daß sogar die Wirkung von etwaigen, sehr kurzzeitigen Störungen, deren Dauer in der Größenordnung der Dauer eines einzelnen Differenzimpulses liegt oder deren Dauer noch kleiner ist, ausgeschaltet wird, da eindurch eine so kurzzeitige Störung verursachter Anteil, der bei einem einzelnen Differenzimpuls groß sein kann, durch die Amplitudenintegration von mehreren Differenzimpulsen gering gemacht wird.

Es sei nun Aufbau und Funktionsweise des übergeordneten Reglers 33 erläutert:

Der übergeordnete Regler 33 umfaßt eine Detektionseinrichtung 44, welche das Vorhandensein äußerer Kräfte ermittelt; sowie eine Verstelleinrichtung 45, welche den Rotor 1 beim Auftreten äußerer Kräfte aus seiner in Axialrichtung stabilisierten Mittenlage in eine axial außermittige Stabilisierungslage verstellt und eine Vergleichseinrichtung 46, welche den Energiebedarf der Rotorlagerung bei in Axialrichtung in der stabilisierten Mittenlage befindlichem Rotor 1 mit dem Energiebedarf der Rotorlagerung in dem Fall vergleicht, in dem sich der Rotor 1 in Axialrichtung in einer außermittigen Stabilisierungslage befindet.

Im einzelnen kann die Detektionseinrichtung 44 beispiels weise ein Strommeßeinrichtung sein, die den elektrischen Strom mißt, der durch die Elektromagnete 19a und 19b fließt. Infolgedessen ist in der Darstellung der Fig. 1 die Detektionseinrichtung über Leitungen 47a und 47b an den Ausgang des Endverstärkers 32 bzw. an die Elektromagnete 19a und 19b angeschlossen. Die Detektionseinrichtung 44 kann alternativ auch eine Strommeßeinrichtung sein, welche den elektrischen Strom mißt, der durch den gesamten Regelkreis fließt, welcher die Lagesensoranordnung, den übrigen Teil der Regelschaltung und die Elektromagnetanordnung umfaßt. Eine weitere Alternative besteht darin, daß die Detektionseinrichtung 44 einen Beschleunigungssensor umfaßt, der die axiale Orientierung oder allgemein die Richtung einer jeweiligen äußeren Kraft detektiert.

Die Detektionseinrichtung 44 ist im vorliegenden Fall über die Vergleichseinrichtung 46 mit der Verstelleinrichtung 45 verbunden, weil sie die äußeren Kräfte durch Ermittlung des Energiebedarfs der Rotorlagerung detektiert und damit nicht nur den Energiebedarf der Rotorlagerung in der axial stabilisierten Mittenlage des Rotors sondern auch den der außermittigen Axialstabilisierungslage des Rotors ermittelt und der Vergleichseinrichtung 46 die Größen liefert, die in der Vergleichseinrichtung miteinander verglichen werden. Wenn dagegen die Detektionseinrichtung 44 die äußeren Kräfte unmittelbar feststellt, beispielsweise durch einen Beschleunigungssensor, ohne den Energiebedarf der Rotorlagerung zu ermitteln, dann kann sie direkt mit der Verstelleinrichtung 45 verbunden sein, wobei in diesem Falle zusätzlich eine weitere Detektionseinrichtung zur Ermittlung des jeweiligen Energiebedarfs bzw. einer diesem Energiebedarf proportionalen Größe vorgesehen ist, welche der Vergleichseinrichtung 46 die miteinander zu vergleichenden Energiebedarfswerte der Rotorlagerung liefern.

Im letzteren Falle ist die Verstelleinrichtung 45 eine sowohl auf das Ausgangssignal der Detektionseinrichtung 44 als auch auf das Ausgangssignal der Vergleicheinrichtung 46 ansprechende Steuereinrichtung, die an ihrem Ausgang ein Signal, beispielsweise eine Spannung abgibt, wodurch über eine Leitung 48, die zum Vorverstärker 30 oder zum Regelverstärker 31 führt, der Sollwert für die axiale Position des Rotors 1 modifiziert wird, und zwar entweder direkt oder indirekt dadurch, daß zu dem am Istwert-Eingang 22 erhaltenen Istwert eine diesen modifizierenden Größe addiert oder subtrahiert wird, was einer Sollwert-Verschiebung entspricht. In der Schaltung nach Fig. 1 ist die Verstelleinrichtung 45 nur mit dem Ausgang der Vergleichseinrichtung 46 verbunden, weil die Detektionseinrichtung 44 nicht nur das Vorhandensein äußerer Kräfte schlechthin sondern vielmehr den Energiebedarf der Rotorlagerung ermitelt, so daß eine Erhöhung dieses Energiebedarfs durch die Vergleichseinrichtung festgestellt und ein entsprechendes Ausgangssignal von letzterer an die Verstelleinrichtung abgegeben wird, welches die Verstelleinrichtung 45 veranlaßt, den Rotor axial zu verschieben. In jedem Falle erfolgt die axiale Verschiebung des Rotors durch eine Vergrößerung oder Verminderung des durch die Elektromagnete 19a und 19b fließenden Stroms infolge der Sollwert-Modifizierung.

Die Vergleichseinrichtung 46 kann beispielsweise eine Abtast- und Halteschaltung umfassen, welche die Energie- bzw. Leistungsbedarfswerte der Elektromagneten 19a und 19b sowie des gesamten Regelkreises, die im vorliegenden Fall durch die Detektionseinrichtung 44 ermittelt werden, in verschiedenen axial veränderten Positionen des Rotors 1 vergleicht und aufgrund dieses Vergleichs ein Ausgangssignal an die Verstelleinrichtung 45 abgibt, das diese veranlaßt, die axiale Position des Rotors 1 im Sinne niedrigerer Energie- bzw. Leistungsbedarfswerte der Elektromagnete 19a und 19b zu verschieben. Die Vergleichseinrichtung 46 besitzt zu diesem Zweck einen Speicher, der den bisherigen Energie- bzw. Leistungsbedarf speichert, damit er mit dem neuen Energie- bzw. Leistungsbedarf verglichen werden kann.

Die Einstellung der axialen Position des Rotors 1 kann auf diese Weise schrittweise durch aufeinanderfolgende Energie- und Leistungsbedarfsvergleiche und durch aufeinanderfolgende Modifizierungen des Sollwerts für die axiale Rotorposition um jeweils kleine Beträge, bis der Energie- bzw. Leistungsbedarf sein Minimum erreicht hat, erfolgen.

Denn der Zweck des übergeordneten Reglers 33 besteht darin, den Sollwert der Rotorposition entlang des durch die Elektromagnete stabilisierten Freiheitsgrades, also vorliegend in axialer Richtung, beim Auftreten langfristig wirkender äußerer Kräfte so zu variieren, daß der Energieverbrauch der Lageregelung bzw. der magnetischen Lagestabilisierung des Rotors 1 vermindert, vorzugsweise minimalisiert wird.

Wie weiter oben im allgemeinen Beschreibungsteil dargelegt worden ist, kann dadurch dieser Energieverbrauch um etwa den Faktor 100 bis 150 vermindert werden.

Die allgemeine Verfahrensweise, mit der das geschieht und die in der Anordnung nach der Fig. 1 verwirktlicht wird, ist zusammenfassend folgende:

(a) Es wird das Vorhandensein äußerer Kräfte detektiert;

(b) der Rotor wird aus seiner stabilisierten Mittenlage in eine neue Stabilisierungslage verlagert; und

(c) es wird detektiert, ob die neue Stabilisierungslage einen geringeren Energiebedarf für die magnetische Lagerung des Rotors zur Folge hat; dieser geringere Energiebedarf ergibt sich dann, wenn die neue Stabilisierungslage derart ist, daß die Permanentmagnete, welche außer den Elektromagneten zur magnetischen Rotorlagerung vorgesehen sind, eine Gegenkraft zu der detekierten äußeren Kraft erzeugen;

(d) die Stabilisierungslage des Rotors wird solange verändert, bis sich ein verminderter, vorzugsweise minimalisierter, Energiebedarf der Rotorlagerung ergibt.

In dem obigen Verfahrensschritt (b) kann der Rotor, anstatt daß er im ersten Schritt mit begrenzter Schrittweite willkürlich in irgendeiner Richtung aus seiner stabilisierten Mittenlage verschoben wird, auch gleich im ersten Schritt im Sinne einer Verminderung des Energiebedarfs verschoben werden, sofern nicht nur das, Vorhandensein der äußeren Kraft sondern auch deren Richtung (positiv oder negativ) in Richtung des Freiheitsgrades, welcher durch die Elektromagnete stabilisiert wird, durch die Detektionseinrichtung festgestellt wird. Wenn die Detektionseinrichtung darüberhinaus auch die Größe der in Richtung des erwähnten Freiheitsgrades auftretenden äußeren Kraft ermittelt, kann eine schrittweise Änderung der Stabilisierungsposition des Rotors entfallen, und der Rotor kann programmiert entsprechend der Richtung und Größe der Kraftkomponente, die entlang des stabilisierten Freiheitsgrades wirkt, in einem Schritt in seine neue Stabilisierungsposition verschoben werden, die einem minimalen Energiebedarf der magnetischen Rotorlagerung entspricht.

Es sei anhand des oberen Teils der Figur 1 und anhand der Figuren 5 und 6 der Drehantrieb des Rotors 1 näher erläutert:

Dieser Drehantrieb kann an sich in verschiedenster Weise ausgebildet sein, zum Beispiel als Wirbelstromantrieb, insbesondere mittels eines Asynchronmotors, als Synchronmotor, als elektronisch kommutierter Elektromotor o. dgl. Bevorzugt ist als Antrieb des Rotors 1 ein Asynchronmotor vorgesehen, der die ringförmigen Antriebselektromagnete 10a, 10b (Fig. 1) oder 10 (Fig. 6) im Stator 5 umfaßt, sowie den Rotor 1 als Kurzschlußläufer (Fig. 1) oder einen Kurzschlußläuferring 10c (Fig. 6) und die dreiphasig betriebenen Antriebselektromagnete 10a, 10b bzw. den Antriebselektromagnet 10, deren Wicklungen die Funktion von Felderregungsspulen haben. Diese Wicklungen, die in Fig. 1 nicht gezeigt und in Fig. 6 bei 105 angedeutet sind, sind vorzugsweise in Sternschaltung mit einer Phasenverschiebung von $\propto = 60°$ geschaltet. Die Spannungsverläufe 49b und 49c, welche zu den Wicklungen der Antriebsmagnete 10a 10b oder 10 führen, folgen den in Figur 1 an diesen Zuführungsleitungen angegebenen Ausdrücken, worin A die maximale Spannungsamplitude, f die Frequenz der Wechselspannung und $\propto$ die vorher erwähnte Phasenverschiebung der drei Phasen 1, 2 und 3 auf den Zuführungsleitungen 49a, 49b und 49c gegeneinander bedeuten.

Als Betriebsspannungsquelle ist ein Oszilator 50 vorgesehen an den die Zuführungsleitungen 49 a, 49 b und 49 c über Leitungen 51 a, 51 b und 51 c und je einen in seinem Verstärkungsgrad steuerbaren Leistungsverstärker 52 a, 52 b und 52 c angeschlossen sind, der die vom Oszilator 50 gelieferte Wechselspannung verstärkt. Der Verstärkungsgrad, und damit die Ausgangsamplitude A der am Ausgang der Leistungsverstärker 52 a, 52 b und 52 c erhaltenen Wechselspannung wird mittels einer über die Leitung 53 auf die Verstärkungsgrad-Steuereingänge gegebenen Steuerspannung gesteuert. Auch die Frequenz des Oszilators 50 ist veränderbar und kann über eine entsprechende Steuerspannung eingestellt werden, die auf den Frequenz-Steuereingang 55 des Oszilators 50 gegeben wird. Auf diese Weise sind die Frequenz f und die Ausgangsspannungsamplitude A des Oszilators, die an den Wicklungen der Antriebselektromagneten 10 a und 10 b bzw. 10 wirksam werden, veränderbar.

Diese Veränderung der Frequenz und der Ausgangsspannugsamplitude erfolgt in vorbestimmter bzw. programmierter Weise mittels eines ersten Charakteristikspeichers 56, dessen Ausgang an den Frequenz-Steuereingang 55 des Oszilators 50 angekoppelt ist, sowie mittels eines zweiten Charakteristikspeichers 57, dessen Ausgang an die Verstärkungsgrad-Steuereingänge 54 a, 54 b und 54 c der Leistungsverstärker angekoppelt ist. Der Eingang des zweiten Charakteristikspeichers 57 ist über einen Frequenz-zu-Spannung-Umsetzer 58 mit einem Ausgang des Oszilators 50 verbunden. Auf diese Weise wird die Amplitude A in Abhängigkeit von der jeweiligen Betriebsfrequenz f, die der Oszilator 50 erzeugt nach einer vorbestimmten Charakteristik, die symbolisch auf dem Charakteristikspeicher 57 angedeutet ist, verändert.

Zur Steuerung der Frequenz f des Oszillators 50 für den ersten Charakteristikspeicher 56 wird auf den Eingang 59 des letzteren ein Steuersignal gegeben, das an sich auf verschiedenste Weise erzeugt werden kann. Im vorliegenden Falle der Steuerung bzw. Regelung des Antriebs einer Blutpumpe, nämlich der Zentrifugalblutpumpe 2, wird dieses Steuersignal von dem Drucksensor 12 erzeugt und über eine Leitung 60 zugeführt. Dieser Drucksensor liefert als Steuersignal eine den Druck auf der venösen Seite oder der Saugseite der Blutpumpe repräsentierende Ausgangsspannung. Durch den ersten Charakteristikspeicher 56 wird diese Ausgangsspannung gemäß einer, Frank-Starling-Charakteristik in eine entsprechend Steuerspannung für den Oszilator 50 derart umgewandelt, daß die Pumpcharakteristik der als künstliches Herz dienenden Zentrifugalblutpumpe 2 den physiologischen Bedingungen entspricht, wie sie von Frank und Starling angegeben worden sind. Hierzu ordnet der Charakteristikspeicher 56 einem vorbestimmten Steuersignal an seinem Eingang 59 eindeutig eine bestimmte Steuerspannung 55 an seinem Ausgang zu, die er, wie erwähnt, auf den Frequenz-Steuereingang des Oszillators 50 gibt.

Der zweite Charakteristikspeicher 57 hat eine solche Charakteristik, daß die auf den Zuführungsleitungen 49a, 49b und 49c erhaltenen Betriebsspannungen in ihrer Frequenz f und ihrer Ausgangsspannungsamplitude A last- und drehzahlabhängig derart verändert werden, daß das vom Asynchronmotor abgegebene Drehmoment, d.h. also vorliegend das am Rotor 1 erzeugte Drehmoment, bei der jeweiligen Drehzahl des Asynchronmotors also vorliegend des Rotors 1, mit dem Lastmoment korreliert.

In einer besonders günstigen und bevorzugten Ausführungsform, die nun anhand der kurzen Darstellung der Figur 5 erläutert sei, ist es so, daß das jeweilige Lastmoment des Rotors auf der Motorkennlinie I bzw. II des Asychronmotors knapp unterhalb des Kippmoments und oberhalb der Kippdrehzahl liegt. In Figur 5 sind zwei ausgewählte Motorkennlinien I und II dargestellt, die jeweils für eine Ausgangsspannungsamplitude $A_1$ bzw. $A_2$ und eine zugeordnete Frequenz $f_1$ bzw. $f_2$ gelten, und zwar gibt in üblicher Weise die Motorkennlinie den Verlauf des Drehmoments M in Abhängigkeit von der Drehzahl N (z.B. in Umdrehungen pro Minute) des Asynchronmotors wieder.

Die Punkte $P_1$ und $P_2$ der erhaltenen Betriebskennlinie B des Motors liegen entsprechend der obigen Definition dieser gewählten Betriebslinie jeweils wenig unterhalb des Kippmoments $M_1$ bzw. $M_2$ und oberhalb der Kippdrehzahl $N_1$ bzw. $N_2$ der für die zugehörige Ausgangsspannungsamplitude $A_1$ bzw. $A_2$ und für die zugehörige Frequenz $f_1$ bzw. $f_2$ gültigen Motorkennlinie I bzw. II. Das gilt für alle anderen Punkte der Betriebskennlinie B des Asynchronmotors.

Auf diese Weise wird erreicht, daß der Rotor 1 der Zentrifugalblutpumpe 2 mit minimalem Energieaufwand angetrieben wird und gleichzeitig die tatsächliche Blutpumpenleistung der Frank-Starling-Charakteristik entspricht, also optimal den physiologischen Bedingungen des menschlichen Blutkreislaufs angepaßt ist. Die zu diesem Zweck vorgesehenen Charakteristikspeicher 56 und 57 können unter Beachtung der Tatsache, daß die jeweilige Charakteristik nichtlinear ist, beispielsweise je eine Widerstandsnetzmatrix oder ein elektronischer Speicher sein.

Die erfindungsgemäße magnetische Rotorlagerung hat insbesondere, jedoch nicht ausschließlich, die folgenden Vorteile:

(a) Sie kommt mit dem gerinsten Energiebedarf aus.

(b) Sie bleibt insbesondere dort, wo sie in sich bewegenden Teilen, Einrichtungen o. dgl. wie z.B. Raketen, vorgesehen ist, trotz der Bewegung der jeweiligen Einrichtung, insbesondere deren Beschleunigung oder Verzögerung, funktionstüchtig, so daß sie ohne Beschränkung in allen Fällen anwendbar ist, in denen der Stator als solcher eine Bewegung erfährt.

(c) Sie ist insbesondere wegen der vorstehend unter (a) und (b) angegebenen Vorteile auch überall dort anwendbar, wo sie mit einem einmaligen, während des Betriebs nicht ergänzbaren Energievorrat auskommen muß, also im sogenannten Inselbetrieb.

**Patentansprüche**

1. Magnetische Rotorlagerung zum berührungslosen Aufhängen eines Rotors, insbesondere des Rotors einer Axial- oder Radial-Zentrifugalblutpumpe, mit einer den Rotor (1) in einem Stator (5), insbesondere einem Gehäuse, aufhängenden und lagestabilisierenden Permanent- und Elektromagnetanordung (17a, 17b, 18a, 18b, 18c, 18d, 19a, 19b), sowie mit einer an wenigstens einen Lagesensor (8a, 8b) bzw. dessen Lagesensorbetriebsschaltung (9) für den Rotor (1) angeschlossenen Regelschaltung (7); wobei die Permanent- und Elektromagnetanordnung (17a, 17b, 18a, 18b, 18c, 18d, 19a, 19b) so ausgebildet ist, daß

(a) der Rotor (1) mittels einer Permanentmagnetanordnung (17a, 17b, 18a, 18b, 18c, 18d), welche wenigstens einen im Rotor (1) und wenigstens einen im Stator (5) angebrachten axial magnetisierten Permanentmagneten (18a, 18b, 18c, 18d bzw. 17a, 17b) umfaßt, bis auf einen einzigen geometrischen Verstellfreiheitsgrad stabil berührungslos im Stator (5) aufgehängt ist;

(b) die Rotorposition nur innerhalb des mittels der Permanentmagnetanordnung (17a, 17b, 18a, 18b, 18c, 18d) nicht stabilisierten geometrischen Verstellfreiheitsgrads mittels einer im Stator (5) vorgesehenen, wenigstens einen axialen Elektromagneten (19a, 19b) umfassenden Elektromagnetanordnung und einer damit zusammenwirkenden, im Rotor (1) befindlichen und wenigstens einen axial magnetisierten Permanentmagneten (18a, 18b, 18c, 18d) umfassenden Permanentmagnetanordnung stabilisiert ist; und

(c) die mit der Elektromagnetanordnung (19a, 19b) zusammenwirkende Permanentmagnetanordnung (18a, 18b, 18c, 18d) wenigstens einen Permanentmagneten (18a, 18b, 18c, 18d) der zum Aufhängen des Rotors (1) vorgesehenen Permanentmagnetanordnung (17a, 17b, 18a, 18b, 18c, 18d) umfaßt, insbesondere aus den im Rotor (1) angebrachten Permanentmagneten (18a, 18b, 18c, 18d) dieser Permanentmagnetanordnung (17a, 17b, 18a, 18b, 18c, 18d) besteht;

dadurch gekennzeichnet, daß der im Rotor (1) angebrachte Permanentmagnet (18a, 18b, 18c, 18d) der Permanentmagnetanordnung (17a, 17b, 18a, 18b, 18c, 18d), mittels deren der Rotor (1) im Stator (5) aufgehängt ist, bezüglich des zugeordneten und im Stator (5) angebrachten Permanentmagneten (17a, 17b) dieser Permanentmagnetanordnung (17a, 17b, 18a, 18b, 18c, 18d) axial derart versetzt angeordnet ist, daß gleichsinnige Magnetpole (S, N) dieser beiden Permanentmagneten (17a, 18a; 17b, 18b; 17a, 18c; 17b, 18d) einander zugewandt sind, wobei die Regelschaltung (7) die Rotorposition in einer Lage stabilisiert, in welcher der absolute Energiebedarf der Elektromagnetanordnung (19a, 19b) ohne äußere Kräfte minimalisiert ist.

2. Magnetische Rotorlagerung nach Anspruch 1, dadurch gekennzeichnet, daß der im Rotor (1) angebrachte Permanentmagnet (18a, 18b, 18c, 18d) der Permanentmagnetanordnung (17a, 17b, 18a, 18b, 18c, 18d), mittels deren der Rotor (1) im Stator (5) aufgehängt ist, bezüglich des zugeordneten und im Stator (5) angebrachten Permanentmagneten (17a, 17b) dieser Permanentmagnetanordnung (17a, 17b, 18a, 18b, 18c, 18d) derart axial versetzt angeordnet ist, daß sich der im Rotor (1) angebrachte Permanentmagnet (18a, 18b, 18c, 18d) gerade außerhalb der axialen Erstreckung des im Stator (5) angebrachten Permanentmagneten (17a, 17b) befindet.

3. Magnetische Rotorlagerung nach Anspruch 1 oder 2, wobei

(a) die den Rotor (1) aufhängende Permanentmagnetanordnung (17a, 17b, 18a, 18b, 18c, 18d) einen konzentrisch zur Rotorachse (16) angeordneten ortsfesten Ringpermanentmagneten (17a, 17b) und einen konzentrisch zu demselben auf der Rotorachse (16) angeordneten Stabpermanentmagneten (18a, 18b) oder Ringpermanentmagneten (18c, 18d) umfaßt; und

(b) die Regelschaltung (7) mit ihrem Istwert-Eingang (22) an den die axiale Lage des Rotors ermittelnden Lagesensor (8a, 8b) bzw. dessen Lagesensorbetriebsschaltung (9) angeschlossen ist;

dadurch gekennzeichnet, daß der Stabpermanentmagnet (18a, 18b) oder der Ringpermanentmagnet (18c, 18d) des Rotors (1) axial innerhalb des Elektromagneten (19a, 19b) angeordnet ist.

4. Magnetische Rotorlagerung nach Anspruch 1, 2 oder 3, dadurch gekennzeichnet, daß die Regelschaltung (7) einen stromproportional regelnden Endverstärker (32) aufweist.

5. Magnetische Rotorlagerung nach Anspruch 1, 2, 3 oder 4, dadurch gekennzeichnet, daß die Regelschaltung (7) eine deren Sollwert beim langzeitigen Einwirken wesentlicher äußerer Kräfte direkt oder durch Veränderung des Istwerts des Lagesensors (8a, 8b) indirekt verschiebenden, den Energieverbrauch der magnetischen Rotorlagerung vermindernden, vorzugsweise minimalisierenden, übergeordneten Regler (33) aufweist.

6. Magnetische Rotorlagerung nach Anspruch 5, dadurch gekennzeichnet, daß der übergeordnete Regler (33) folgendes umfaßt:

(a) eine das Vorhandensein äußerer Kräfte ermittelnde Detektionseinrichtung (44);

(b) eine den Rotor (1) aus seiner stabilisierten Mittenlage in eine außermittige Stabilisierungslage verstellende Verstelleinrichtung (45); und

(c) eine den Energiebedarf der Rotorlagerung in der stabilisierten Mittenlage mit deren Energiebedarf in der außermittigen Stabilisierungslage oder den Energiebedarf in zwei verschiedenen außermittigen Stabilisierungslagen vergleichenden Vergleichseinrichtung (46).

7. Magnetische Rotorlagerung nach Anspruch 6, dadurch gekennzeichnet, daß die Detektionseinrichtung (44) eine Strommeßeinrichtung zum Messen des durch die Elektromagnetanordnung (19a, 19b) fließenden elektrischen Stromes ist oder eine Strommeßeinrichtung zum Messen des gesamten elektrischen Stromes, welcher durch den den Lagesensor bzw. die Lagesensoren (8a, 8b) die Regelschaltung (7) und die Elektromagnetanordnung (19a, 19b) umfassenden Regelkreis fließt.

8. Magnetische Rotorlagerung nach Anspruch 6, dadurch gekennzeichnet, daß die Verstelleinrichtung (45) eine Lagesensor-Sollwertveränderungseinrichtung ist.

9. Magnetische Rotorlagerung nach Anspruch 6, dadurch gekennzeichnet, daß die Vergleichseinrichtung (46) eine Strommeßeinrichtung zum Messen des durch die Elektromagnetanordnung (19a, 19b) oder durch den gesamten Regelkreis fließenden elektrischen Stromes und eine die Strommeßwerte speichernde und vergleichende Sample- und Holdschaltung umfaßt.

10. Magnetische Rotorlagerung nach einem der Ansprüche 1 bis 9, dadurch gekennzeichnet, daß der Lagesensor wenigstens eine von der Rotorwelle betätigte Impulsstrahlungsschranke (8a, 8b) mit einem impulsgesteuerten Strahlungssendeelement (24a, 24b) und einem Strahlungsempfangselement (29a, 29b) umfaßt.

11. Magnetische Rotorlagerung nach Anspruch 10, dadurch gekennzeichnet, daß zwei Lagesensoren (8a, 8b) und eine daran angekoppelte Lagesensorbetriebsschaltung (9) vorgesehen sind und folgendes umfassen:

(a) zwei Impulsstrahlungsschranken (8a, 8b), von denen je eine mit je einem axialen Ende (23a, 23b) der Rotorwelle zusammenwirkt;

(b) eine die Ausgangssignale der Strahlungsempfangselemente (29a, 29b) der beiden Impulsstrahlungsschranken (8a, 8b) substrahierende Subtraktionseinrichtung (38);

(c) eine die Ausgangssignale der Subtraktionseinrichtung (38) integrierende Integrationseinrichtung (39);

(d) eine an den Ausgang der Integrationseinrichtung (39) angeschlossene Sample- und Holdschaltung (40); und

(e) eine Ablaufsteuereinrichtung (42) zur Steuerung des Schließ- und Öffnungstakts und der Schließ- und Öffnungszeiten eines ersten, zweiten und dritten Schalters (S$_1$, S$_2$, S$_3$), von denen der erste Schalter (S$_1$) zwischen dem Ausgang der Subtraktionseinrichtung (38) und dem Eingang der Integrationseinrichtung (39) vorgesehen ist, während der zweite Schalter (S$_2$) ein die Rückstellung der Integrationseinrichtung (39) bewirkender Schalter ist und der dritte Schalter (S$_3$) zwischen dem Ausgang der Integrationseinrichtung (39) und dem Eingang der Sample- und Holdschaltung (40) angeordnet ist, wobei die Ablaufsteuereinrichtung (42) die Schließzeiten der drei Schalter (S$_1$, S$_2$, S$_3$) derart steuert, daß der erste Schalter (S$_1$) immer dann angeschlossen ist, wenn die Strahlungssendeelemente (24a, 24b) senden, während das Schließen des dritten Schalters (S$_3$) jeweils nach Integration einer vorbestimmten Anzahl von Ausgangssignalen der Subtraktionseinrichtung (38) erfolgt und der zweite Schalter (S$_2$) nach Öffnen des dritten Schalters (S$_3$) geschlossen wird.

12. Magnetische Rotorlagerung nach Anspruch 10 oder 11, dadurch gekennzeichnet, daß die Impulsstrahlungsschranken (8a, 8b) Infrarotlichtschranken oder Ultraschallschranken sind.

13. Magnetische Rotorlagerung nach einem der Ansprüche 1 bis 12, dadurch gekennzeichnet, daß als Antrieb des Rotors (1) ein Asynchronmotor (1, 10a, 10b, 10, 10c) vorgesehen ist, der an einen Oszillator (50) als Betriebsspannungsquelle angeschlossen ist, dessen Frequenz (f) und Ausgangsspannungsamplitude (A) last- und drehzahlabhängig derart veränderbar sind, daß das vom Asynchronmotor (1, 10a, 10b, 10, 10c) abgegebene Drehmoment (M) bei der jeweiligen Drehzahl (N) des Asynchronmotors (1, 10a, 10b, 10, 10c) mit dem Lastmoment korreliert, insbesondere das jeweilige Lastmoment des Rotors (1) auf der Motorkennlinie (I, II) des Asynchronmotors (1, 10a, 10b, 10, 10c) wenig unterhalb des Kippmoments ($M_1$, $M_2$) und oberhalb der Kippdrehzahl ($N_1$, $N_2$) liegt.

14. Magnetische Rotorlagerung nach einem der Ansprüche 1 bis 13, dadurch gekennzeichnet, daß bei Anordnung des Rotors (1) als Förderorgan in einer Blutpumpe, insbesondere einer Zentrifugalblutpumpe, (2) eine Antriebsleistungssteuereinrichtung vorgesehen ist, die folgendes umfaßt:

(a) einen Drucksensor (12) auf der Saugseite der Blutpumpe (2);

(b) einen mit seinem Eingang an den Drucksensor (12) angeschlossenen und einer vorbestimmten Eingangsgröße eine bestimmte Ausgangsgröße zuordnenden und abgebenden Charakteristikspeicher (56); und

(c) eine die tatsächliche Blutpumpenleistung entsprechend der Ausgangsgröße des Charakteristikspeichers (56) steuernde Leistungssteuereinrichtung, vorzugsweise einen an einen die Blutpumpe (2) antreibenden Asynchronmotor als Betriebsspannungsquelle angeschlossenen und in seiner Frequenz und Ausgangsspannung last- und drehzahlabhängig veränderbaren Oszillator (50).

15. Magnetische Rotorlagerung nach Anspruch 14, dadurch gekennzeichnet, daß ein die Betriebsspannungsamplitude (A) des Asynchronmotors in Abhängigkeit von der Frequenz (f) des Oszillators (50) steuernder weiterer Charakteristikspeicher (57) vorgesehen ist.

## Claims

1. A magnetic rotor bearing system for contactless suspension of a rotor, more particularly the rotor of an axial or radial centrifugal blood pump, comprising an arrangement (17a, 17b, 18a, 18b, 18c, 18d, 19a, 19b) of permanent magnets and electromagnets which suspend and stabilize the position of the rotor (1) in a stator (5), more particularly a casing, and a control circuit (7) connected to at least one sensor (8a, 8b) of the position of the rotor (1) or a circuit (9) for operating a sensor, the permanent magnet and the electromagnet arrangement (17a, 17b, 18a, 18b, 18c, 18d, 19a, 19b) being designed so that

(a) The rotor (1) is suspended without contact in the stator (5) and so as to be stable except for a single degree of freedom of geometrical adjustment, by means of a permanent magnet arrangement (17a, 17b, 18a, 18b, 18c, 18d) comprising at least one axially magnetised permanent magnet in the rotor (1) and at least one in the stator (5) (18a, 18b, 18c, 18d or 17a, 17b respectively);

(b) The rotor position is stabilised, but only within the degree of freedom of geometrical adjustment not stabilised by the permanent-magnet arrangement (17a, 17b, 18a, 18b, 18c, 18d), by an electromagnet arrangement comprising at least one axial electromagnet (19a, 19b) in the stator (5) and a cooperating permanent-magnet arrangement disposed in the rotor (1) and comprising at least one axially magnetised permanent magnet (18a, 18b, 18c, 18d); and

(c) The permanent magnet arrangement (18a, 18b, 18c, 18d) co-operating with the electromagnet arrangement (19a, 19b) comprises at least one permanent magnet (18a, 18b, 18c, 18d) out of the permanent-magnet arrangement (17a, 17b, 18a, 18b, 18c, 18d) for suspending the rotor (1), more particularly from among those permanent magnets (18a, 18b, 18c, 18d) of the permanent-magnet arrangement (17a, 17b, 18a, 18b, 18c, 18d) which are disposed in the rotor (1);

characterised in that that permanent magnet (18a, 18b, 18c, 18d) out of the permanent-magnet arrangement (17a, 17b, 18a, 18b, 18c, 18d) disposed in the rotor (1) and used for suspending the rotor (1) in the stator (5) is axially offset relative to the associated permanent magnet (17a, 17b) out of the permanent-magnet arrangement (17a, 17b, 18a, 18b, 18c, 18d) and disposed in the stator (5), so that like poles (S, N) of the two permanent magnets (17a, 18a; 17b, 18b; 17a, 18c; 17b, 18d) are facing one another, and the control circuit (7) stabilises the rotor in a position in which the absolute energy required by the electromagnet arrangement (19a, 19b) without external forces is at a minimum.

2. A magnetic rotor bearing system according to claim 1, characterised in that the permanent magnet (18a, 18b, 18c, 18d) in the permanent-magnet arrangement (17a, 17b, 18a, 18b, 18c, 18d) which is disposed in the rotor (1) and used for suspending the rotor (1) in the stator (5), is axially offset relative to the associated permanent magnet (17a, 17b) out of the permanent-magnet arrangement (17a, 17b, 18a, 18b, 18c, 18d) disposed in the stator (5) so that the permanent magnet (18a, 18b, 18c, 18d) in the rotor (1) lies just outside the axial extension of the permanent magnet (17a, 17b) in the stator (5).

3. A magnetic rotor bearing system according to claim 1 or 2, in which

(a) The permanent magnet arrangement (17a, 17b, 18a, 18b, 18c, 18d) suspending the rotor (1) comprises a stationary annular permanent magnet (17a, 17b) disposed concentrically with the rotor axis (16) and a permanent bar magnet (18a, 18b) or permanent annular magnet (18c, 18d) disposed on the rotor axis (16) so as to be concentric with the first-mentioned magnet, and

(b) The actual-value input (22) of the control circuit (7) is connected to the sensor (8a, 8b) for determining the rotor position, or to the circuit (9) for driving the sensor;

characterised in that the permanent bar magnet

17

(18a, 18b) or the permanent annular magnet (18c, 18d) of the rotor (1) is disposed axially inside the electromagnet (19a, 19b).

4. A magnetic rotor bearing system according to claim 1, 2 or 3, characterised in that the control circuit (7) comprises a final amplifier (32) which exerts control in proportion to the current.

5. A magnetic rotor bearing system according to claim 1, 2, 3 or 4, characterised in that the control circuit (7) comprises an overriding controller (33) which, after prolonged action of considerable external forces, alters the set value of the control circuit, either directly or indirectly by altering the actual value of the position sensor (8a, 8b), and which reduces and preferably minimizes the energy consumption of the magnetic rotor bearing system.

6. A magnetic rotor bearing system according to claim 5, characterised in that the overriding controller (33) comprises the following:

(a) A detector (44) for detecting the presence of external forces;

(b) An adjuster (45) which moves the rotor (1) from its stabilised central position into an eccentric stabilisation position; and

(c) A comparator (46) which compares the energy required by the rotor bearing system in the stabilised central position with the energy required in the eccentric stabilisation position or the energy required in two different eccentric stabilisation positions.

7. A magnetic rotor bearing system according to claim 6, characterised in that the detector (44) is an ammeter for measuring the electric current flowing through the electromagnet arrangement (19a, 19b), or an ammeter for measuring the total electric current flowing through the control circuit comprising the position sensor or position sensors (8a, 8b), the control circuit (7), and the electromagnet arrangement (19a, 19b).

8. A magnetic rotor bearing system according to claim 6, characterised in that the adjuster (45) is a means for altering the set value of the position sensor.

9. A magnetic rotor bearing system according to claim 6, characterised in that the comparator comprises an ammeter for measuring the electric current flowing through the electromagnet arrangement (19a, 19b) or the entire control circuit, and a sample and hold circuit which stores and compares the measurements of the current.

10. A magnetic rotor bearing system according to any of claims 1 to 9, characterised in that the position sensor comprises at least one pulse radiation barrier (8a, 8b) actuated by the rotor shaft and comprising a pulse-controlled radiation transmitter (24a, 24b) and a radiation receiver (29a, 29b).

11. A magnetic rotor bearing system according to claim 10, characterised in that two position sensors (8a, 8b) and a position-sensor operating circuit (9) coupled thereto are provided and comprise the following:

(a) Two pulse radiation barriers (8a, 8b), each co-operating with a respective end (23a, 23b) of the rotor shaft;

(b) A subtractor (38) which subtracts the output signals of the radiation receivers (29a, 29b) of the two pulse radiation barriers (8a, 8b);

(c) An integrator (39) which integrates the output signals from the subtractor (38);

(d) A sample and hold circuit (40) connected to the input of the integrator (39); and

(e) A process control device (42) for controlling the closing and opening cycle and the closing and opening times of a first, second and third switch ($S_1$, $S_2$, $S_3$), the first switch ($S_1$) being disposed between the output of the subtractor (38) and the input of the integrator (39), the second switch ($S_2$) being used to reset the integrator (39), and the third switch ($S_3$) being disposed between the output of the integrator (39) and the input of the sample and hold circuit (40), and the process control device (42) controls the closing times of the three switches ($S_1$, $S_2$, $S_3$) so that the first switch ($S_1$) is always connected when the radiation transmitters (24a, 24b) are transmitting, whereas the third switch ($S_3$) is closed whenever a predetermined number of output signals from the subtractor (38) have been integrated, and the second switch ($S_2$) is closed after the third switch ($S_3$) opens.

12. A magnetic rotor bearing system according to claim 10 or II, characterised in that the pulse-radiation barriers (8a, 8b) are infrared light barriers or ultrasonic barriers.

13. A magnetic rotor bearing system according to any of claims 1 to 12, characterised in that the rotor (1) is driven by an asynchronous motor (1, 10a, 10b, 10, 10c) connected to an oscillator (50) which is a source of operating voltage and has a frequency (f) and an output voltage (A) which can be directly varied in dependence on the load and speed so that the torque (M) delivered by the asynchronous motor (1, 10a, 10b, 10, 10c) at the instantaneous speed (N) of the asynchronous motor (1, 10a, 10b, 10, 10c), when correlated with the moment of load, more particularly the respective moment of load of the rotor (1), lies slightly below the pull-out torque ($M_1$, $M_2$) and above the tilting speed ($N_1$, $N_2$) on the characteristic (I, II) of the asynchronous motor (1, 10a, IDb, 10, 10c).

14. A magnetic rotor bearing system according to any of claims 1 to 13, characterised in that when the rotor (1) is disposed at a conveying means in a blood pump, more particularly a centrifugal blood pump (2), a drive-power control device is provided and comprises the following:

(a) A pressure sensor (12) on the suction side of the blood pump (2);

(b) A characteristic store (56) which has its input connected to the pressure sensor (12) and which delivers a given output variable after assigning it to a predetermined input variable, and

(c) A power control device which controls the actual blood pump power in dependence on the output variable from the characteristic store (56), the control device preferably being an oscillator (50) connected to a source of operating voltage, i.e. an asynchronous motor driving the blood pump (2), and having a frequency and an output voltage which are variable in dependence on the load and speed.

15. A magnetic rotor bearing system according to claim 14, characterised in that an additional characteristic store (57) is provided and controls the operating voltage (A) of the asynchronous motor in dependence on the frequency (f) of the oscillator (50).

## Revendications

1. Système de suspension magnétique de rotor pour la suspension sans contact d'un rotor, en particulier du rotor d'une pompe à sang centrifuge axiale ou radiale, comportant un ensemble d'aimants permanents et d'électro-aimants (17a, 17b, 18a, 18b, 18c, 18d, 19a, 19b) assurant la suspension et la stabilisation de position du rotor (1) dans un stator (5), en particulier dans un carter, ainsi qu'un circuit de régulation (7) raccordé à au moins un capteur de position (8a, 8b) ou son circuit d'activation du capteur de position (9) pour le rotor (1), l'ensemble d'aimants permanents et d'électro-aimants (17a, 17b, 18a, 18b, 18c, 18d, 19a, 19b) étant réalisé de telle façon que

a) le rotor (1) est, au moyen d'un ensemble d'aimants permanents (17a, 17b, 18a, 18b, 18c, 18d) qui comprend au moins un aimant permanent (18a, 18b, 18c, 18d) aimanté axialement placé dans le rotor (1) et au moins un aimant permanent aimanté axialement (17a, 17b) placé dans le stator (5), suspendu dans le stator (5) sans contact de façon stable jusqu'à un seul degré de liberté de déplacement géométrique;

b) la position du rotor est, seulement à l'intérieur du degré de liberté de déplacement géométrique non stabilisé au moyen de l'ensemble d'aimants permanents (17a, 17b, 18a, 18b, 18c, 18d), stabilisé au moyen d'un ensemble d'électro-aimants prévu dans le stator (5), comprenant au moins un électro-aimant axial (19a, 19b) et un ensemble d'aimants permanents coopérant avec lui, se trouvant dans le rotor (1) et comprenant au moins un aimant permanent aimanté axialement (18a, 18b, 18c, 18d); et

c) l'ensemble d'aimants permanents (18a, 18b, 18c, 18d) coopérant avec l'ensemble d'électro-aimants (19a, 19b) comprend au moins un aimant permanent (18a, 18b, 18c, 18d) de l'ensemble d'aimants permanents (17a, 17b, 18a, 18b, 18c, 18d) prévu pour la suspension du rotor (1), en particulier parmi les aimants permanents (18a, 18b, 18c, 18d) placés dans le rotor (1) de cet ensemble d'aimants permanents (17a, 17b, 18a, 18b, 18c, 18d);

caractérisé en ce que l'aimant permanent (18a, 18b, 18c, 18d), placé dans le rotor (1), de l'ensemble d'aimants permanents (17a, 17b, 18a, 18b, 18c, 18d) au moyen duquel le rotor (1) est suspendu dans le stator (5) est disposé avec un décalage axial par rapport à l'aimant permanent (17a, 17b), correspondant et placé dans le stator (5), de cet ensemble d'aimants permanents (17a, 17b, 18a, 18b, 18c, 18d) de telle façon que des pôles magnétiques (S, N) de même sens de ces deux aimants permanents (17a, 18a, 17b, 18b, 17a, 18c, 17b, 18d) sont en regard, le circuit de régulation (7) stabilisant la position du rotor dans une position dans laquelle la consommation absolue d'énergie de l'ensemble d'électro-aimants (19a, 19b) est minimisée sans forces extérieures.

2. Système de suspension magnétique de rotor selon la revendication 1, caractérisé en ce que l'aimant permanent (18a, 18b, 18c, 18d), placé dans le rotor (1), de l'ensemble d'aimants permanents (17a, 17b, 18a, 18b, 18c, 18d) au moyen duquel le rotor (1) est en suspension dans le stator (5), est décalé axialement par rapport à l'aimant permanent (17a, 17b), correspondant et placé dans le stator (5), de cet ensemble d'aimants permanents (17a, 17b, 18a, 18b, 18c, 18d) de telle façon que l'aimant permanent (18a, 18b, 18c, 18d) de telle façon que l'aimant permanent (18a, 18b, 18c, 18d) placé dans le rotor (1) se trouve juste à l'extérieur de l'etendue axiale de l'aimant permanent (17a, 17b) placé dans le stator (5).

3. Système de suspension magnétique de rotor selon la revendication 1 ou 2, dans lequel:

a) l'ensemble d'aimants permanents (17a, 17b, 18a, 18b, 18c, 18d) assurant la suspension du rotor (1) comprend un aimant permanent annulaire fixe (17a, 17b) placé concentriquement par rapport à l'axe (16) du rotor et un aimant permanent (18a, 18b) en forme de barreau ou un aimant permanent annulaire (18c, 18d) disposé concentriquement par rapport à celui-ci sur l'axe (16) du rotor; et

b) le circuit de régulation (7) est raccordé, par son entrée de valeurs réelles (22) au capteur de position (8a, 8b) déterminant la position axiale du rotor ou à son circuit d'activation de capteur de position (9);

caractérisé en ce que l'aimant permanent en forme de barreau (18a, 18b) ou l'aimant permanent annulaire (18c, 18d) du rotor (1) est disposé axialement à l'intérieur de l'électro-aimant (19a, 19b).

4. Système de suspension magnétique de rotor selon la revendication 1, 2 ou 3, caractérisé en ce que le circuit de régulation (7) comporte un amplificateur terminal (32) à réglage proportionnel de l'intensité.

5. Système de suspension magnétique de rotor selon la revendication 1, 2, 3 ou 4, caractérisé en ce que le circuit de régulation (7) comporte un régulateur (33) hiérarchiquement supérieur décalant sa valeur de consigne en cas d'action de longue durée de forces extérieures importantes, directement ou indirectement par variation de la valeur réelle du capteur de position (8a, 8b), diminuant, de préférence minimisant, la consommation d'énergie du système de suspension magnétique de rotor.

6. Système de suspension magnétique de rotor selon la revendication 5, caractérisé en ce que le régulateur hiérarchiquement supérieur (33) comprend les composants suivants:

a) un dispositif de détection (44) déterminant la présence de forces extérieures;

b) un dispositif de déplacement (45) faisant passer le rotor (1) de sa position centrale stabilisée à une position de stabilisation décentrée; et

c) un dispositif comparateur (46) comparant la consommation d'énergie de la suspension de rotor dans la position centrale stabilisée à sa consommation d'énergie dans la position de stabilisation décentrée, ou la consommation d'énergie dans deux positions de stabilisation décentrées différentes.

7. Système de suspension magnétique de rotor selon la revendication 6, caractérisé en ce que le dispositif de détection (44) est un dispositif de mesure d'intensité destiné à mesurer le courant électrique passant par l'ensemble d'électro-aimants (19a, 19b) ou un dispositif de mesure d'intensité destiné à mesurer l'intensité électrique totale qui passe par le circuit de régulation comprenant le capteur de position ou les capteurs de position (8a, 8b), le circuit de régulation (7) et l'ensemble d'électro-aimants (19a, 19b).

8. Suspension magnétique de rotor selon la revendication 6, caractérisée en ce que le dispositif de déplacement (45) est un dispositif de modification de la valeur de consigne du capteur de position.

9. Système de suspension magnétique de rotor selon la revendication 6, caractérisé en ce que le dispositif de comparaison (46) comprend un dispositif de mesure de l'intensité pour mesurer le courant électrique passant par l'ensemble d'électro-aimants (19a, 19b) ou l'ensemble du circuit de régulation et un circuit d'échantillonnage et de maintien mémorisant et comparant les valeurs de mesure d'intensité.

10. Système de suspension magnétique de rotor selon l'une des revendications 1 à 9, caractérisé en ce que le capteur de position comprend au moins un barrage à rayonnement à impulsions (8a, 8b) comportant un élément émetteur de rayonnement (24a, 24b) commandé par impulsions et un élément récepteur de rayonnement (29a, 29b).

11. Système de suspension magnétique de rotor selon la revendication 10, caractérisé en ce que deux capteurs de position (8a, 8b) et un circuit d'activation de capteurs de position (9) qui leur est couplé sont prévus et comportent les composants suivants:

a) deux barrages à rayonnement à impulsions (8a, 8b) dont chacun coopère avec une extrémité axiale respective (23a, 23b) de l'arbre du rotor;

b) un dispositif de soustraction (38) soustrayant les signaux de sortie des éléments récepteurs de rayonnement (29a, 29b) des deux barrages à rayonnement à impulsions (8a, 8b);

c) dispositif d'intégration (39) intégrant les signaux de sortie du dispositif de soustraction (38);

d) un circuit d'échantillonnage et de maintien (40) raccordé à la sortie du dispositif d'intégration (39); et

e) un dispositif de commande de processus (42) pour commander le rythme de fermeture et d'ouverture et les instants de fermeture et d'ouverture d'un premier, d'un second et d'un troisième interrupteur (S1, S2, S3) dont le premier interrupteur (S1) est prévu entre la sortie du dispositif de soustraction (38) et l'entrée du dispositif d'intégration (39), tandis que le second interrupteur (S2) est un interrupteur provoquant la remise à zéro du dispositif d'intégration (39) et le troisième interrupteur (S3) est placé entre la sortie du dispositif d'intégration (39) et l'entrée du circuit d'échantillonnage et de maintien (40), le dispositif de commande de processus (42) commandant les instants de fermeture des trois interrupteurs (S1, S2, S3) de telle façon que le premier interrupteur (S1) soit toujours fermé lorsque les éléments émetteurs de rayonnement (24a, 24b) émettent, tandis que la fermeture du troisième interrupteur (S3) a lieu chaque fois après l'intégration d'un nombre prédéterminé de signaux de sortie du dispositif de soustraction (38) et que le second interrupteur (S2) se ferme après l'ouverture du troisième interrupteur (S3).

12. Système de suspension magnétique de rotor selon la revendication 10 ou 11, caractérisé en ce que les barrages à rayonnement à impulsions (8a, 8b) sont des barrages photoélectriques à infrarouge ou des barrages à ultrasons.

13. Système de suspension magnétique de rotor selon l'une des revendications 1 à 12, caractérisé en ce qu'on prévoit, comme mécanisme d'entraînement du rotor (1), un moteur asynchrone (1, 10a, 10b, 10, 10c) qui est raccordé à un oscillateur (50) constituant une source de tension de service dont la fréquence (f) et l'amplitude de tension de sortie (A) sont variables en fonction de la charge et de la vitesse de rotation de telle façon que le couple (M) fourni par le moteur asynchrone (1, 10a, 10b, 10, 10c) est, pour chaque vitesse de rotation (N) du moteur asynchrone (1, 10a, 10b, 10, 10c), en corrélation avec le couple résistant, en particulier chaque couple résistant du rotor (1) se trouve sur la caractéristique moteur (I, II) du moteur asynchrone (1, 10a, 10b, 10, 10c) un peu au-dessous du couple maximum (M1, M2) et au-dessus de la vitesse de rotation maximale (N1, N2).

14. Système de suspension magnétique de rotor selon l'une des revendications 1 à 13, caractérisé en ce que, lorsque le rotor (1) est placé, en tant qu'organe de transport, dans une pompe à sang, en particulier dans une pompe à sang centrifuge (2), on prévoit un dispositif de commande de puissance motrice qui comprend les composants suivants:

a) un capteur de pression (12) du côté d'aspiration de la pompe à sang (2);

b) une mémoire de caractéristiques (56) reliée par son entrée au capteur de pression (12) et rattachant à une grandeur d'entrée prédéterminée une grandeur de sortie déterminée et la fournissant; et

c) un dispositif de commande de puissance commandant la puissance effective de la pompe à sang selon la grandeur de sortie de la mémoire de caractéristiques (56), de préférence un oscillateur (50) relié à un moteur asynchrone entraînant la pompe à sang (2) constituant une source de tension de service et dont la fréquence et la tension de sortie peuvent varier en fonction de la charge et de la vitesse de rotation.

15. Suspension magnétique de rotor selon la revendication 14, caractérisé en ce qu'on prévoit une autre mémoire de caractéristiques (57) commandant l'amplitude (A) de la tension de service du moteur asynchrone en fonction de la fréquence (f) de l'oscillateur (50).

# FIG. 1

FIG. 2

FIG. 3

2

FIG. 4a

FIG. 4b

FIG. 5

# FIG. 6

EP 0 150 320 B1